(19) Europäisches Patentamt / European Patent Office / Office européen des brevets

(11) EP 4 527 852 A1

(12) **EUROPEAN PATENT APPLICATION**
published in accordance with Art. 153(4) EPC

(43) Date of publication:
**26.03.2025 Bulletin 2025/13**

(21) Application number: **23806923.1**

(22) Date of filing: **16.05.2023**

(51) International Patent Classification (IPC):
***C07K 16/28*** *(2006.01)* ***C12N 15/13*** *(2006.01)*
***A61K 39/395*** *(2006.01)* ***A61K 47/68*** *(2017.01)*
***A61K 31/4745*** *(2006.01)* ***A61P 35/00*** *(2006.01)*

(52) Cooperative Patent Classification (CPC):
**A61K 47/68037; A61K 9/0019; A61K 9/19; A61K 31/4745; A61K 39/39591; A61K 47/183; A61K 47/26; A61K 47/68; A61K 47/6855; A61P 35/00; C07K 16/2803;** C07K 2317/94

(86) International application number:
**PCT/CN2023/094502**

(87) International publication number:
**WO 2023/221971 (23.11.2023 Gazette 2023/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **16.05.2022 CN 202210530522**

(71) Applicants:
- **Jiangsu Hengrui Pharmaceuticals Co., Ltd.**
  **Lianyungang, Jiangsu 222047 (CN)**
- **Shanghai Hengrui Pharmaceutical Co., Ltd.**
  **Shanghai 200245 (CN)**

(72) Inventors:
- **SHAN, Shuang**
  **Shanghai 200245 (CN)**
- **TIAN, Chenmin**
  **Shanghai 200245 (CN)**

(74) Representative: **Dragotti & Associati S.R.L.**
**Via Nino Bixio, 7**
**20129 Milano (IT)**

Remarks:
The complete document including Reference Table(s) and the Sequence Listing(s) can be downloaded from the EPO website

(54) **PHARMACEUTICAL COMPOSITION CONTAINING ANTI-NECTIN-4 ANTIBODY DRUG CONJUGATE AND USE THEREOF**

(57) Provided are a pharmaceutical composition containing an anti-Nectin-4 antibody drug conjugate and a use thereof. The provided pharmaceutical composition has good stability.

EP 4 527 852 A1

## Description

The present application claims priority to Chinese Patent Application No. CN202210530522.7 filed on May 16, 2022.

## TECHNICAL FIELD

The present disclosure belongs to the field of pharmaceutical formulations and particularly relates to a pharmaceutical composition comprising an anti-Nectin-4 antibody-drug conjugate and use thereof as an anti-cancer medicament.

## BACKGROUND

The statements herein merely provide background information related to the present disclosure and may not necessarily constitute the prior art.

Nectin-4 (PVRL4) is a 66 kDa type I transmembrane glycoprotein and belongs to the Nectin family of the Ig superfamily. It plays a key role in various biological processes (proliferation, differentiation and migration) of epithelial, endothelial, immune and neural cells. Research shows that Nectin-4 plays an important role in the development, infiltration and metastasis of malignant tumor tissues such as bladder cancer, breast cancer and lung cancer. This role is associated with the activation of Cdc42 and Rac and the alteration of cytoskeletal actin and causes the proliferation, infiltration and metastasis of tumor cells.

Nectin-4 is a tumor-specifically expressed antigen. It is expressed in 50% of bladder cancer, 49% of breast cancer and 86% of lung cancer and is frequently found in tumors with poor prognosis, but its expression is not found in most normal tissues. The above information suggests that Nectin-4 can be an ideal target for tumor treatment.

Enfortumab vedotin (Padcev) is a Nectin-4-targeting conjugate in which MMAE is conjugated. More antibody-drug conjugates taking Nectin-4 as a target have great significance as anti-tumor drugs for research.

ADCs are of more complicated heterostructures than antibodies, and therefore, a greater challenge has been posed to ADC formulations for therapeutic purposes.

## SUMMARY

The present disclosure provides a pharmaceutical composition comprising an antibody-drug conjugate and a buffer, wherein the antibody-drug conjugate has a structure represented by formula NEC49-9-A:

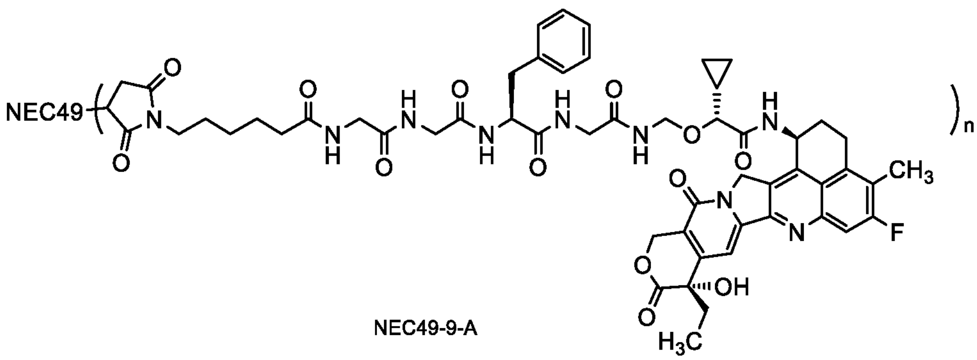

NEC49-9-A

;

wherein:

NEC49 is an anti-Nectin-4 antibody comprising a heavy chain HCDR1, a heavy chain HCDR2, and a heavy chain HCDR3 set forth in SEQ ID NO: 8, SEQ ID NO: 9, and SEQ ID NO: 10; and a light chain LCDR1, a light chain LCDR2, and a light chain LCDR3 set forth in SEQ ID NO: 11, SEQ ID NO: 12, and SEQ ID NO: 13; preferably a heavy chain variable region set forth in SEQ ID NO: 4 and a light chain variable region set forth in SEQ ID NO: 5; and more preferably a heavy chain set forth in SEQ ID NO: 6 and a light chain set forth in SEQ ID NO: 7;
n is a decimal or integer from 1 to 10, preferably from 1 to 8, and more preferably from 3 to 5;
the buffer is histidine-histidine hydrochloride buffer;
the pharmaceutical composition has a pH of about 5.0 to about 6.5, preferably a pH of about 5.5 to about 6.5, and more preferably a pH of about 5.9 to about 6.2.

The present disclosure provides a pharmaceutical composition comprising an antibody-drug conjugate and a buffer, wherein the antibody-drug conjugate has a structure represented by formula NEC49-9-A:

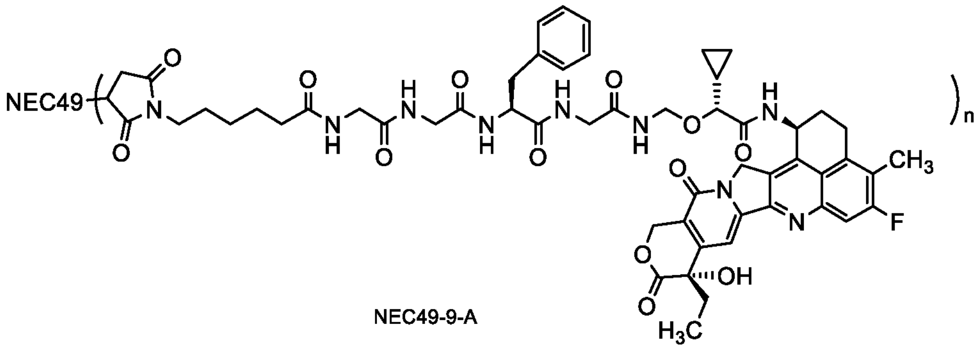

NEC49-9-A

;

wherein:

NEC49 is an anti-Nectin-4 antibody derived from the antibody NEC49 of the PCT application PCT/CN2022/089129, comprising a heavy chain variable region set forth in SEQ ID NO: 4 and a light chain variable region set forth in SEQ ID NO: 5;

n is a decimal or integer from 1 to 10, preferably from 1 to 8, more preferably from 3 to 5, and most preferably from about 3.5 to 4.7;

the buffer is histidine-histidine hydrochloride buffer, and the composition has a pH of about 5.0 to about 6.5, preferably a pH of about 5.5 to about 6.5, and more preferably a pH of about 5.9 to about 6.2.

In an alternative embodiment, the anti-Nectin-4 antibody NEC49 in the pharmaceutical composition comprises a heavy chain set forth in SEQ ID NO:6 (SEQ ID NO: 21 in PCT/CN2022/089129) and a light chain set forth in SEQ ID NO: 7 (SEQ ID NO: 22 in PCT/CN2022/089129):

NEC49 heavy chain:

EVQLLESGGGLVQPGGSLRLSCAASGFTFSSYAMSWVRQAPGKGLEWVSAIYS
GGSTYYADSVKGRFTISRDNSKNTLYLQMNSLRAEDTAVYYCTRHGGDSGSWS
YYYYGMDVWGQGTTVTVSSASTKGPSVFPLAPSSKSTSGGTAALGCLVKDYFP
EPVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK

SEQ ID NO: 6

NEC49 light chain:

DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPLTFGQGTRLEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

SEQ ID NO: 7.

In an alternative embodiment, the pharmaceutical composition has a pH of about 5.0 to about 6.5, non-limiting

examples including about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, and any range between these point values; preferably a pH of about 5.5 to about 6.5, and more preferably a pH of about 5.9 to about 6.2.

In an alternative embodiment, the concentration of the buffer in the pharmaceutical composition is about 5 mM to about 50 mM, non-limiting examples including 5 mM, 10 mM, 12 mM, 15 mM, 16 mM, 17 mM, 18 mM, 19 mM, 20 mM, 25 mM, 26 mM, 27 mM, 28 mM, 29 mM, 30 mM, 31 mM, 32 mM, 33 mM, 34 mM, 35 mM, 40 mM, 45 mM, 50 mM, and any range between these point values, preferably about 10 mM to about 50 mM, more preferably about 20 mM to about 40 mM; and most preferably about 30 mM.

In an alternative embodiment, the pharmaceutical composition further comprises a surfactant. The surfactant may be selected from the group consisting of polysorbate, polysorbate 20, polysorbate 80, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymer of ethylene and propylene glycol, and the like. The preferred surfactant is polysorbate 80 or polysorbate 20, and the more preferred surfactant is polysorbate 80.

In an alternative embodiment, the concentration of the surfactant in the pharmaceutical composition is about 0.01 mg/mL to about 1.0 mg/mL, preferably about 0.05 mg/mL to about 0.6 mg/mL, further preferably about 0.05 mg/mL to about 0.4 mg/mL, more preferably about 0.1 mg/mL to about 0.3 mg/mL, or about 0.2 mg/mL to about 0.6 mg/mL, and most preferably about 0.2 mg/mL, non-limiting examples including about 0.01 mg/mL, 0.02 mg/mL, 0.05 mg/mL, 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL, 0.6 mg/mL, 0.7 mg/mL, 0.8 mg/mL, 0.9 mg/mL, 1.0 mg/mL, and any range between these point values.

In an alternative embodiment, the aforementioned pharmaceutical composition further comprises a sugar. "Sugar" of the present disclosure includes the general composition $(CH_2O)_n$ and derivatives thereof, including monosaccharides, disaccharides, trisaccharides, polysaccharides, sugar alcohols, reducing sugars, non-reducing sugars, and the like. The sugar may be selected from the group consisting of glucose, sucrose, trehalose, α,α-trehalose dihydrate, lactose, fructose, maltose, dextran, glycerin, erythritol, glycerol, arabitol, sylitol, sorbitol, mannitol, melibiose, melezitose, raffinose, mannotriose, stachyose, maltose, lactulose, maltulose, glucitol, maltitol, lactitol, iso-maltulose, and the like. The preferred sugar is non-reducing disaccharide, the more preferred sugar is selected from the group consisting of α,α-trehalose and sucrose, and the most preferred sugar is sucrose.

In an alternative embodiment, the concentration of the sugar in the aforementioned pharmaceutical composition is about 25 mg/mL to about 80 mg/mL, preferably about 30 mg/mL to about 50 mg/mL, non-limiting examples including 25 mg/mL, 30 mg/mL, 35 mg/mL, 40 mg/mL, 45 mg/mL, 50 mg/mL, 55 mg/mL, 60 mg/mL, 70 mg/mL, 80 mg/mL, and any range between these point values, and preferably 40 mg/mL.

In an alternative embodiment, the aforementioned pharmaceutical composition further comprises an amino acid, and the amino acid is preferably glycine.

In an alternative embodiment, the concentration of the amino acid in the aforementioned pharmaceutical composition is about 6 mg/mL to about 15 mg/mL, about 7 mg/mL to about 11 mg/mL, preferably about 7 mg/mL to about 10 mg/mL, more preferably about 7 mg/mL to about 9 mg/mL, about 8 mg/mL to about 9 mg/mL, non-limiting examples including about 6 mg/mL, 6.5 mg/mL, 7 mg/mL, 7.2 mg/mL, 7.6 mg/mL, 7.8 mg/mL, 8 mg/mL, 8.5 mg/mL, 9 mg/mL, 9.5 mg/mL, 10 mg/mL, 10.2 mg/mL, 11 mg/mL, 12 mg/mL, 13 mg/mL, 14 mg/mL, 15 mg/mL, and any range between these point values, and most preferably about 9 mg/mL.

In an alternative embodiment, the concentration of the glycine in the aforementioned pharmaceutical composition is about 6 mg/mL to about 15 mg/mL, about 7 mg/mL to about 11 mg/mL, preferably about 7 mg/mL to about 10 mg/mL, more preferably about 7 mg/mL to about 9 mg/mL, about 8 mg/mL to about 9 mg/mL, non-limiting examples including about 6 mg/mL, 6.5 mg/mL, 7 mg/mL, 7.2 mg/mL, 7.6 mg/mL, 7.8 mg/mL, 8 mg/mL, 8.5 mg/mL, 9 mg/mL, 9.5 mg/mL, 10 mg/mL, 10.2 mg/mL, 11 mg/mL, 12 mg/mL, 13 mg/mL, 14 mg/mL, 15 mg/mL, and any range between these point values, and most preferably about 9 mg/mL.

In an alternative embodiment, the concentration of the antibody-drug conjugate in the pharmaceutical composition is about 1 mg/mL to about 100 mg/mL on a protein concentration basis, non-limiting examples including 1 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, 40 mg/mL, 50 mg/mL, 60 mg/mL, 70 mg/mL, 80 mg/mL, 90 mg/mL, 100 mg/mL, and any range between these point values; preferably, the concentration of the antibody-drug conjugate is about 10 mg/mL to about 30 mg/mL on a protein concentration basis; more preferably, the concentration of the antibody-drug conjugate is about 18 mg/mL to about 22 mg/mL on a protein concentration basis; most preferably, the concentration of the antibody-drug conjugate is about 20 mg/mL. Specifically,

non-limiting examples include 20.1 mg/mL, 20.2 mg/mL, 20.3 mg/mL, 20.4 mg/mL, 20.5 mg/mL, 20.6 mg/mL, 20.7 mg/mL, 20.8 mg/mL, 20.81 mg/mL, 20.82 mg/mL, 20.83 mg/mL, 20.84 mg/mL, 20.85 mg/mL, 20.86 mg/mL, 20.87 mg/mL, 20.88 mg/mL, 20.89 mg/mL, 20.9 mg/mL, 20.91 mg/mL, 20.92 mg/mL, 20.93 mg/mL, 20.94 mg/mL, 20.95 mg/mL, 20.96 mg/mL, 20.97 mg/mL, 20.98 mg/mL, 20.99 mg/mL, 21 mg/mL, and any range between these point values. The "on a protein concentration basis" refers to on the basis of the concentration of the antibody moiety in the antibody-drug conjugate.

In an alternative embodiment, the range of drug loading (n) may be the average number of cytotoxic drugs bound per anti-Nectin-4 antibody, non-limiting examples including the case that the average number of cytotoxic drugs bound per antibody is about 1, 2, 3, 4, 5, 6, 7, 8, 9, 10, 11, 12, and any range between these point values. Preferably, the range of drug loading (n) is selected from the group consisting of 1-10, 1-8, 2-8, 2-7, 2-6, 2-5, 2-4, 3-4, 3-5, 3.5-4.7, 5-6, 5-7, 5-8, and 6-8. Illustratively, the drug loading (n) may be an average of 1, 2, 3, 4, 5, 6, 7, 8, 9, or 10. n is a decimal or integer.

In an alternative embodiment, the drug loading (n) is about 3.5-4.7.

In an alternative embodiment, the drug loading (n) is about 4.

In an alternative embodiment, the pharmaceutical composition comprises:

(a) on a protein concentration basis, about 1 mg/mL to about 100 mg/mL said antibody-drug conjugate, (b) about 0.05 mg/mL to about 0.4 mg/mL polysorbate, (c) about 30 mg/mL to about 50 mg/mL sucrose or $\alpha,\alpha$-trehalose dihydrate, (d) about 6 mg/mL to about 15 mg/mL glycine, and (e) about 10 mM to about 50 mM histidine-histidine hydrochloride buffer; the pharmaceutical composition having a pH of about 5.5 to 6.5.

In an alternative embodiment, the pharmaceutical composition comprises:

(a) on a protein concentration basis, about 10 mg/mL to about 30 mg/mL said antibody-drug conjugate, (b) about 0.1 mg/mL to about 0.3 mg/mL polysorbate, (c) about 30 mg/mL to about 50 mg/mL sucrose or $\alpha,\alpha$-trehalose dihydrate, (d) about 7 mg/mL to about 11 mg/mL glycine, and (e) about 10 mM to about 50 mM histidine-histidine hydrochloride buffer; the pharmaceutical composition having a pH of about 5.5 to 6.5.

In an alternative embodiment, the pharmaceutical composition comprises:

(a) on a protein concentration basis, about 10 mg/mL to about 30 mg/mL said antibody-drug conjugate, (b) about 0.1 mg/mL to about 0.3 mg/mL polysorbate 80, (c) about 30 mg/mL to about 50 mg/mL sucrose, (d) about 7 mg/mL-11 mg/mL glycine, and (e) about 10 mM to about 50 mM histidine-histidine hydrochloride buffer; the pharmaceutical composition having a pH of about 5.5-6.5.

In an alternative embodiment, the pharmaceutical composition comprises:

(a) on a protein concentration basis, about 10 mg/mL to about 30 mg/mL said antibody-drug conjugate, (b) about 0.1 mg/mL to about 0.3 mg/mL polysorbate 80, (c) about 30 mg/mL to about 50 mg/mL sucrose, (d) about 7 mg/mL-11 mg/mL glycine, and (e) about 20 mM to about 40 mM histidine-histidine hydrochloride buffer; the pharmaceutical composition having a pH of about 5.5-6.5.

In an alternative embodiment, the pharmaceutical composition comprises:

(a) on a protein concentration basis, about 18 mg/mL to about 22 mg/mL said antibody-drug conjugate, (b) about 0.2 mg/mL polysorbate 80, (c) about 40 mg/mL sucrose, (d) about 9 mg/mL glycine, and (e) about 30 mM histidine-histidine hydrochloride buffer; the pharmaceutical composition having a pH of about 5.9-6.2.

In an alternative embodiment, the pharmaceutical composition comprises:

(a) on a protein concentration basis, about 20 mg/mL said antibody-drug conjugate, (b) about 0.2 mg/mL polysorbate 80, (c) about 40 mg/mL sucrose, (d) about 9 mg/mL glycine, and (e) about 30 mM histidine-histidine hydrochloride buffer; the pharmaceutical composition having a pH of about 6.0.

In an alternative embodiment, the pharmaceutical composition according to any one of the foregoing is a liquid formulation. The liquid formulation or the reconstituted formulation of the present disclosure has relatively good stability. Further, stable liquid formulations include liquid formulations that exhibit desirable features after storage at temperatures including 40 °C for 1 month.

The present disclosure also provides a lyophilized formulation comprising an antibody-drug conjugate, wherein the formulation can be reconstituted to form the pharmaceutical composition described above.

The present disclosure also provides a method for preparing a lyophilized formulation comprising an antibody-drug conjugate, the method comprising a step of lyophilizing the pharmaceutical composition described above.

In an alternative embodiment, the lyophilizing in the method for preparing a lyophilized formulation comprising an antibody-drug conjugate comprises steps of pre-freezing, primary drying, and secondary drying in sequence. The lyophilizing is performed by freezing the formulation and subsequently sublimating the water at a temperature suitable for primary drying. Under these conditions, the product is at a temperature lower than the eutectic point or the collapse temperature of the formulation. Typically, the temperature for the primary drying ranges from about -30 °C to 25 °C (assuming the product remains frozen during the primary drying). The formulation, the size and type of the container (e.g., glass vial) containing the sample, and the liquid volume determine the time required for drying, which may range from a few hours to several days (e.g., 40-60 hours). The secondary drying may be performed at about 0 °C-40 °C, mainly depending on the type and size of the container and the type of the protein used. The time needed for the secondary drying is determined by the desired residual water content in the product, and it typically takes at least about 5 hours. Generally, the

water content of the formulation lyophilized at low pressure is less than about 5%, preferably less than about 3%. The pressure may be the same as that applied to the step of primary drying; preferably, the pressure of the secondary drying is lower than that of the primary drying. The conditions for the lyophilizing may vary depending on the formulation and the vial size.

In an alternative example of the present disclosure, 4.4 mL of the composition stock solution is lyophilized through a procedure as follows: pre-freezing at temperatures of 5 °C and -45 °C in sequence, primary drying at a temperature of -20 °C with a degree of vacuum of 10 Pa, and secondary drying at a temperature of 25 °C with degrees of vacuum of 10 Pa and 1 Pa in sequence.

In some embodiments, the lyophilized formulation is stable at 40 °C for at least 7 days, at least 14 days, at least 28 days, or at least 30 days.

The present disclosure also provides a lyophilized formulation comprising an antibody-drug conjugate obtained by lyophilizing the pharmaceutical composition of the antibody-drug conjugate described above.

The present disclosure also provides a reconstituted solution comprising an antibody-drug conjugate prepared and obtained by reconstituting the lyophilized formulation described above.

The present disclosure also provides a method for preparing the reconstituted solution described above comprising a step of reconstituting the aforementioned lyophilized formulation with a solution selected from the group consisting of, but not limited to, water for injection, normal saline, or glucose solution.

In an alternative embodiment, the aforementioned reconstituted solution comprises:

(a) on a protein concentration basis, about 1 mg/mL to about 100 mg/mL said antibody-drug conjugate, (b) about 0.05 mg/mL to about 0.4 mg/mL polysorbate, (c) about 30 mg/mL to about 50 mg/mL sucrose or $\alpha,\alpha$-trehalose dihydrate, (d) about 6 mg/mL to about 15 mg/mL glycine, and (e) about 10 mM to about 50 mM histidine-histidine hydrochloride buffer; the pharmaceutical composition having a pH of about 5.5 to 6.5.

In an alternative embodiment, the aforementioned reconstituted solution comprises:

(a) on a protein concentration basis, about 10 mg/mL to about 30 mg/mL said antibody-drug conjugate, (b) about 0.1 mg/mL to about 0.3 mg/mL polysorbate, (c) about 30 mg/mL to about 50 mg/mL sucrose or $\alpha,\alpha$-trehalose dihydrate, (d) about 7 mg/mL to about 11 mg/mL glycine, and (e) about 10 mM to about 50 mM histidine-histidine hydrochloride buffer; the pharmaceutical composition having a pH of about 5.5 to 6.5.

In an alternative embodiment, the aforementioned reconstituted solution comprises:

(a) on a protein concentration basis, about 10 mg/mL to about 30 mg/mL said antibody-drug conjugate, (b) about 0.1 mg/mL to about 0.3 mg/mL polysorbate 80, (c) about 30 mg/mL to about 50 mg/mL sucrose, (d) about 7 mg/mL to about 11 mg/mL glycine, and (e) about 10 mM to about 50 mM histidine-histidine hydrochloride buffer; the pharmaceutical composition having a pH of about 5.5 to 6.5.

In an alternative embodiment, the aforementioned reconstituted solution comprises:

(a) on a protein concentration basis, about 10 mg/mL to about 30 mg/mL said antibody-drug conjugate, (b) about 0.1 mg/mL to about 0.3 mg/mL polysorbate 80, (c) about 30 mg/mL to about 50 mg/mL sucrose, (d) about 7 mg/mL to about 11 mg/mL glycine, and (e) about 20 mM to about 40 mM histidine-histidine hydrochloride buffer; the pharmaceutical composition having a pH of about 5.5 to 6.5.

In an alternative embodiment, the aforementioned reconstituted solution comprises:

(a) about 18 mg/mL to about 22 mg/mL said antibody-drug conjugate, (b) about 0.2 mg/mL polysorbate 80, (c) about 40 mg/mL sucrose, (d) about 9 mg/mL glycine, and (e) about 30 mM histidine-histidine hydrochloride; the pharmaceutical composition having a pH of about 5.9 to 6.2.

In an alternative embodiment, the aforementioned reconstituted solution comprises:

(a) about 20 mg/mL said antibody-drug conjugate, (b) about 0.2 mg/mL polysorbate 80, (c) about 40 mg/mL sucrose, (d) about 9 mg/mL glycine, and (e) about 30 mM histidine-histidine hydrochloride; the pharmaceutical composition having a pH of about 6.0. The present disclosure also provides a kit comprising a container containing the pharmaceutical composition, the lyophilized formulation, or the reconstituted solution described above. In some embodiments, the container is a tubular injection vial made of neutral borosilicate glass.

The disclosure also provides use of the aforementioned pharmaceutical composition or lyophilized formulation or reconstituted solution in the preparation of a medicament for treating or preventing a tumor.

The present disclosure also provides a method for treating a disease comprising administering to a subject in need thereof a therapeutically or prophylactically effective amount of the aforementioned pharmaceutical composition or lyophilized formulation or reconstituted solution or kit.

The present disclosure also provides the aforementioned pharmaceutical composition or lyophilized formulation or reconstituted solution or kit as a medicament for treating or preventing a disease.

The present disclosure also provides the aforementioned pharmaceutical composition or lyophilized formulation or reconstituted solution or kit as a medicament for treating or preventing a tumor.

In an alternative embodiment, the disease or the tumor is a Nectin-4-mediated disease or disorder.

The present disclosure also provides use of the aforementioned pharmaceutical composition or lyophilized

formulation or reconstituted solution or kit as a medicament in the preparation of a medicament for treating a viral infection, a tumor, or a cancer, wherein the tumor or the cancer is selected from the group consisting of urothelial cancer (e.g., metastatic urothelial cancer or locally advanced urothelial cancer), ureter cancer, breast cancer, pancreatic cancer, lung cancer (including non-small cell lung cancer and small cell lung cancer), renal cancer (e.g., renal cell carcinoma), liver cancer (e.g., hepatocellular carcinoma), esophageal cancer (also referred to as "esophageal carcinoma"), a laryngeal tumor, a pharyngeal tumor, an oral tumor, gastric cancer, ovarian cancer, prostate cancer (e.g., metastatic castration-resistant prostate cancer), bladder cancer (locally advanced bladder cancer), colorectal cancer (including colon cancer and rectal cancer), head and neck cancer, squamous cell cancer, and melanoma.

In some embodiments, the pharmaceutical composition or lyophilized formulation or reconstituted solution or kit according to any one of the above is an intravenous injection formulation, a subcutaneous injection formulation, an intraperitoneal injection formulation, or an intramuscular injection formulation. In some embodiments, it is an intravenous injection formulation.

As is well known to those skilled in the art, one, some or all of the features of the various embodiments described in the present disclosure may be further combined to form other embodiments of the present disclosure. The above embodiments of the present disclosure and other embodiments obtained by combination are further illustrated through the following detailed description.

## DETAILED DESCRIPTION

The present disclosure provides a pharmaceutical composition that favors production and administration and is stable in properties. Undesirable instability may include any one or more of aggregation, deamidation (e.g., Asn deamidation), oxidation (e.g., Met oxidation), isomerization (e.g., Asp isomerization), clipping/hydrolysis/fragmentation (e.g., hinge region fragmentation), succinimide formation, unpaired cysteines, dissociation of toxins, and the like. Specifically, the pharmaceutical composition described in the present disclosure comprises an antibody-drug conjugate and a buffer.

### Terminology

In order to facilitate the understanding of the disclosure, some technical and scientific terms are specifically defined below. Unless otherwise explicitly defined herein, all other technical and scientific terms used herein have the meanings generally understood by those of ordinary skill in the art to which the present disclosure belongs.

"Antibody-drug conjugate (ADC)" refers to a conjugate formed by linking an antibody (or an antigen-binding fragment thereof) to a biologically active cytotoxin or a small-molecule drug with cell killing activity by a linker unit.

The term "drug loading" is also referred to as the drug-to-antibody ratio (DAR), which is the average number of drugs conjugated per antibody in the ADC. It may range, for example, from about 1 to about 10 drugs conjugated per antibody, and in certain embodiments, from about 1 to about 8 drugs conjugated per antibody, and is preferably selected from the group consisting of integers or decimals of 1-8, 2-8, 2-7, 2-6, 2-5, 2-4, 3-4, 3-5, 3-6, 4-5, 5-6, 5-7, 5-8, 6-7, and 6-8. Illustratively, the drug loading may be an average of any of 1, 2, 3, 4, 5, 6, 7, 8, 9, and 10. The ADC general formula of the present disclosure includes a set of antibodies conjugated to drugs within a certain range as described above. In an embodiment of the present disclosure, the drug loading may be represented by n. Drug loading can be determined by conventional methods such as UV/visible spectroscopy, mass spectrometry, ELISA assay and HPLC.

The term "linker unit" or "linker fragment" refers to a chemical structure fragment or bond that is linked to an antibody or an antigen-binding fragment thereof at one end and to a drug at the other end, and it may also be linked to a drug after being linked to another linker.

The linker comprises a stretcher, a spacer, and an amino acid unit and may be synthesized using methods known in the art, such as those described in US20050238649A1. The linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers may be used (Chari et al., Cancer Research, 52: 127-131(1992); U.S. Patent No. 5,208,020).

The term "drug linker arm fragment" or "drug-linker fragment" refers to a fragment formed by linking a drug and a linker unit, which can be linked to an antibody or an antigen-binding fragment thereof through the other end of the linker unit.

The loading of the cytotoxic drug can be controlled using the following non-limiting methods, including:

(1) controlling the molar ratio of the linking drug linker arm fragment to the antibody,
(2) controlling reaction time and temperature, and
(3) selecting different reaction reagents.

The three-letter and single-letter codes for amino acids used in the present disclosure are as described in J. Biol. Chem., 243, p3558 (1968).

The "antibody" described in the present disclosure is used in the broadest sense and encompasses a variety of antibody structures, including but not limited to monoclonal antibodies, polyclonal antibodies; monospecific antibodies, multispecific antibodies (e.g., bispecific antibodies), and full-length antibodies, and antibody fragments (or antigen-binding fragments, or antigen-binding moieties), as long as they exhibit the desired antigen-binding activity.

The engineered antibody or antigen-binding fragment of the present disclosure can be prepared and purified by conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems will result in glycosylation of antibodies, particularly at the highly conservative N-terminal site of the Fc region. Positive clones are expanded in a serum-free culture medium of a bioreactor to produce antibodies. The culture medium with the secreted antibody can be purified by conventional techniques. For example, purification is performed using an A or G Sepharose FF column containing an adjusted buffer. Non-specifically bound fractions are washed away. The bound antibody is eluted by the pH gradient method, and the antibody fragments are detected by SDS-PAGE and collected. The antibody can be filtered and concentrated by conventional methods. Soluble mixtures and polymers can also be removed by conventional methods, such as molecular sieves and ion exchange. The resulting products need to be immediately frozen, e.g., at -70 °C, or lyophilized.

"Buffer" refers to a buffer that resists changes in pH by the action of its acid-base conjugate components. Examples of buffers that maintain the pH within an appropriate range include acetate, succinate, gluconate, histidine, oxalate, lactate, phosphate, citrate, tartrate, fumarate, glycylglycine, and other organic acid buffers.

"Histidine buffer" is a buffer comprising histidine. Examples of histidine buffers include histidine-histidine hydrochloride, histidine-histidine acetate, histidine-histidine phosphate, histidine-histidine sulfate, and the like. The histidine-histidine hydrochloride buffer is preferred. The histidine-histidine hydrochloride buffer may be prepared from histidine and hydrochloric acid, or from histidine and histidine hydrochloride.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer that comprises succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared from succinic acid and sodium hydroxide, or from succinic acid and sodium succinate.

"Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The preferred phosphate buffer is disodium hydrogen phosphate-sodium dihydrogen phosphate.

"Acetate buffer" is a buffer comprising acetate ions. Examples of acetate buffers include acetic acid-sodium acetate, histidine-histidine acetate, acetic acid-potassium acetate, acetic acid-calcium acetate, acetic acid-magnesium acetate, and the like. The preferred acetate buffer is acetic acid-sodium acetate.

"Pharmaceutical composition" refers to a mixture containing one or more of the antibody-drug conjugates described herein or physiologically/pharmaceutically acceptable salts or prodrugs thereof, and other chemical components; the other components are, for example, physiologically/pharmaceutically acceptable carriers and excipients. The purpose of the pharmaceutical composition is to maintain the stability of the active ingredient of the antibody and promote the administration to an organism, which facilitates the absorption of the active ingredient, thereby exerting biological activity.

As used herein, "pharmaceutical composition" and "formulation" are not mutually exclusive.

Unless otherwise stated, the solvent in the pharmaceutical composition described herein in solution form is water.

"Lyophilized formulation" refers to a formulation or a pharmaceutical composition obtained by lyophilizing a pharmaceutical composition or a formulation in liquid or solution form in vacuum.

The terms "about" and "approximately" as used herein mean that a numerical value is within an acceptable error range for the specific value determined by those of ordinary skill in the art, and the numerical value depends in part on how the value is measured or determined (i.e., the limits of the measurement system). For example, "about" may mean a standard deviation within 1 or more than 1 in each practice in the art. Or, "about" or "substantially comprising" may mean a range of up to 20%. Furthermore, particularly for biological systems or processes, the term may mean up to an order of magnitude or up to 5-fold of a numerical value. Unless otherwise stated, when a specific value is provided in the present application and claims, the meaning of "about" or "substantially comprise" should be assumed to be within an acceptable error range for that specific value.

The pharmaceutical composition of the present disclosure can achieve a stable effect: a pharmaceutical composition in which the antibody-drug conjugate substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring the stability of a protein or an antibody-drug conjugate, and the stability after storage for a selected period of time at a selected temperature can be measured.

A stable formulation is one in which no significant change is observed under the following conditions: stored at refrigeration temperature (2-8 °C) for at least 3 months, preferably 6 months, and more preferably 1 year. In addition, stable liquid formulations include liquid formulations that exhibit desirable features after storage at temperatures including 25 °C for periods including 1 month, 2 months, or 3 months. Further, stable liquid formulations include liquid formulations that exhibit desirable features after storage at temperatures including 40 °C for periods of time including 10 days, 20 days, and 1 month. Typical examples for stability are as follows: generally, no more than about 10%, preferably no more than about 5%, of antibody monomers aggregate or are degraded as measured by SEC-HPLC. The formulation is a pale yellow, nearly colorless and clear liquid, or colorless, or clear to slightly opalescent, by visual analysis. The concentration, pH, and osmolality of the formulation have a change of no more than $\pm$10%. Generally, a decrease of no more than about 10%, preferably no more than about 5% is observed. Generally, aggregation of no more than about 10%, preferably no more than about 5% is formed.

An antibody-drug conjugate "retains its physical stability" in a pharmaceutical formulation if it shows no significant increase in aggregation, precipitation and/or denaturation upon visual examination of color and/or clarity, or as measured by UV light scattering, size exclusion chromatography (SEC) and dynamic light scattering (DLS). Changes in protein conformation can be evaluated by fluorescence spectroscopy (which determines the protein tertiary structure) and by FTIR spectroscopy (which determines the protein secondary structure).

An antibody-drug conjugate "retains its chemical stability" in a pharmaceutical formulation if it shows no significant chemical change. Chemical stability can be evaluated by detecting and quantifying chemically changed protein. Degradation processes that often change the chemical structure of proteins include hydrolysis or clipping (evaluated by methods such as size exclusion chromatography and CE-SDS), oxidation (evaluated by methods such as peptide mapping in conjunction with mass spectroscopy or MALDI/TOF/MS), deamidation (evaluated by methods such as ion-exchange chromatography, capillary isoelectric focusing, peptide mapping, and isoaspartic acid measurement), and isomerization (evaluated by measuring the isoaspartic acid content, peptide mapping, etc.).

An antibody-drug conjugate "retains its biological activity" in a pharmaceutical formulation if the biological activity of the antibody-drug conjugate at a given time is within a predetermined range of the biological activity exhibited at the time the pharmaceutical formulation was prepared.

"Optional" or "optionally" means that the event or circumstance subsequently described may, but does not necessarily, occur, and this description includes instances where the event or circumstance occurs or does not occur. For example, "optionally comprising 1-3 antibody heavy chain variable regions" means that the antibody heavy chain variable region of a particular sequence may, but does not necessarily, exist.

For the preparation of conventional pharmaceutical compositions, refer to Chinese Pharmacopoeia.

The term "carrier" for the drug of the present disclosure refers to a system that can alter how the drug gets into a human body and the distribution of the drug in the human body, control the release rate of the drug, and deliver the drug to a targeted organ. The drug carrier release and targeted system can reduce drug degradation and loss, reduce side effects, and improve bioavailability. For example, polymeric surfactants that can be used as carriers can self-assemble due to their unique amphiphilic structures to form various forms of aggregates; preferred examples are, e.g., micelles, microemulsions, gels, liquid crystals, and vesicles. The aggregates have the capability of encapsulating drug molecules and have good permeability for membranes, and therefore can be used as excellent drug carriers.

"Administer" and "treat", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids. "Administer" and "treat" can refer to, for example, therapeutic, pharmacokinetic, diagnostic, research, and experimental methods. The treatment of cells comprises contacting a reagent with cells and contacting the reagent with a fluid, where the fluid is in contact with the cells. "Administer" and "treat" also refer to treating, e.g., cells, by reagents, diagnosis, binding compositions or by another cell *in vitro* and *ex vivo.* "Treat", when applied to humans, veterinary, or research subjects, refers to therapeutic treatment, preventive or prophylactic measures, and research and diagnostic applications.

"Treating" or "treatment" refers to administering a therapeutic agent, e.g., a composition comprising any one of the antibody-drug conjugates of the present disclosure, either internally or externally to a patient with one or more disease symptoms on which the therapeutic agent is known to have a therapeutic effect. Typically, the therapeutic agent is administered in an amount effective to alleviate one or more disease symptoms in the patient or population being treated to induce regression of such symptoms or to inhibit the development of such symptoms to any clinically measurable degree.

The amount of the therapeutic agent effective to alleviate any particular disease symptom (also known as a "therapeutically effective amount") may vary depending on a variety of factors, such as the disease state, age, and weight of the patient, and the ability of the drug to produce a desired therapeutic effect in the patient. Whether a disease symptom has been alleviated can be evaluated by any clinical testing methods commonly used by doctors or other health care professionals to evaluate the severity or progression of the symptom. Although the embodiments of the disclosure (for example, treatment methods or kits) may not be effective in alleviating every disease symptom of interest, they shall reduce the disease symptoms of interest in a statistically significant number of patients, as determined according to any statistical testing methods known in the art, such as Student t-test, chi-square test, Mann and Whitney's U test, Kruskal-Wallis test (H test), Jonckheere-Terpstra test and Wilcoxon test.

"Effective amount" comprises an amount sufficient to ameliorate or prevent a symptom or disorder of a medical disease. An effective amount also means an amount sufficient to allow or facilitate diagnosis. The effective amount for a particular patient or veterinary subject may vary depending on factors such as the disorder to be treated, the general health of the patient, the method and route and dose of administration, and the severity of side effects. An effective amount may be the maximum dose or administration regimen to avoid significant side effects or toxic effects.

"Exchange" refers to the exchange of a solvent system that solubilizes an antibody protein or antibody-drug conjugate. For example, a high-salt or hypertonic solvent system comprising the antibody protein or antibody-drug conjugate is exchanged, by physical operations, with a buffer system of a stable formulation, such that the antibody protein or antibody-drug conjugate is present in the stable formulation. The physical operations include, but are not limited to, ultrafiltration, dialysis, or reconstitution following centrifugation.

## Examples

The present disclosure is further described below with reference to examples, which, however, are not intended to limit the scope of the present disclosure. The experimental methods in the examples of the present disclosure in which specific conditions are not specified are generally performed under conventional conditions, for example, by referring to Antibodies: A Laboratory Manual and Molecular Cloning: A Laboratory Manual by Cold Spring Harbor Laboratory, or under conditions recommended by the manufacturer of the raw material or the goods. Reagents without specific origins indicated are commercially available conventional reagents.

## I. Antibody Preparation

### Example 1. Construction of Cell Strains Highly Expressing Nectin-4

PBABE-Nectin4 lentiviral expression vector plasmids, pVSV-G and pGag-pol lentiviral system packaging vectors were transfected into viral packaging cells 293T using Lipofectamine 3000 transfection reagent. The culture medium supernatant containing viruses was collected, filtered, and centrifuged at ultra-high speed. Chinese hamster ovary cells CHO-K1 were allowed to be infected with the concentrated virus, screened using puromycin for two to three weeks, and subjected to FACS single-cell sorting. According to the Nectin-4 expression levels on the surface of CHO-K1 cells infected with lentivirus determined by FACS, monoclonal cell strains highly expressing Nectin-4 were selected, expanded and cryopreserved for later use.

Amino acid sequence of human Nectin-4 (UniProtKB - Q96NY8-1)

MPLSLGAEMWGPEAWLLLLLLLASFTGRCPAGELETSDVVTVVLGQDAKLPCF
YRGDSGEQVGQVAWARVDAGEGAQELALLHSKYGLHVSPAYEGRVEQPPPPRN
PLDGSVLLRNAVQADEGEYECRVSTFPAGSFQARLRLRVLVPPLPSLNPGPALEE
GQGLTLAASCTAEGSPAPSVTWDTEVKGTTSSRSFKHSRSAAVTSEFHLVPSRSM
NGQPLTCVVSHPGLLQDQRITHILHVSFLAEASVRGLEDQNLWHIGREGAMLKC
LSEGQPPPSYNWTRLDGPLPSGVRVDGDTLGFPPLTTEHSGIYVCHVSNEFSSRD
SQVTVDVLDPQEDSGKQVDLVSASVVVVGVIAALLFCLLVVVVVLMSRYHRRK
AQQMTQKYEEELTLTRENSIRRLHSHHTDPRSQPEESVGLRAEGHPDSLKDNSS
CSVMSEEPEGRSYSTLTTVREIETQTELLSPGSGRAEEEEDQDEGIKQAMNHFVQ
ENGTLRAKPTGNGIYINGRGHLV SEQ ID NO: 1.

A positive control antibody EV201 was prepared according to WO2012047724 (page 115). The heavy and light chain amino acid sequences of EV201 (enfortumab vedotin) are as follows:

EV201 heavy chain:

EVQLVESGGGLVQPGGSLRLSCAASGFTFSSYNMNWVRQAPGKGLEWVSYISS
SSSTIYYADSVKGRFTISRDNAKNSLSLQMNSLRDEDTAVYYCARAYYYGMDV
WGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKRV
EPKSCDKTHTCPPCPAPELLGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSREEMTKNQVSLTCLVKGFYPSD
IAVEWESNGQPENNYKTTPPVLDSDGSFFLYSKLTVDKSRWQQGNVFSCSVMH
EALHNHYTQKSLSLSPGK

SEQ ID NO: 2

EV201 light chain:

DIQMTQSPSSVSASVGDRVTITCRASQGISGWLAWYQQKPGKAPKFLIYAASTL
QSGVPSRFSGSGSGTDFTLTISSLQPEDFATYYCQQANSFPPTFGGGTKVEIK
RTVAAPSVFIFPPSDEQLKSGTASVVCLLNNFYPREAKVQWKVDNALQSGNSQE
SVTEQDSKDSTYSLSSTLTLSKADYEKHKVYACEVTHQGLSSPVTKSFNRGEC

SEQ ID NO: 3.

**Example 2. Screening for Anti-Human Nectin-4 Monoclonal Antibody**

The NEC49 in PCT/CN2022/089129 is cited as the antibody of the present disclosure. PCT/CN2022/089129 is incorporated herein by reference in its entirety. A positive clone was obtained by panning using a fully human semi-synthetic phage antibody library and antigen Biotinylated Human Nectin-4 (purchased from Beijing ACROBiosystems Biotech Ltd., Cat. # NE4-H82E7) followed by phage detection by ELISA. The positive clone was sequenced. After the sequence was obtained, the positive clone was inserted into the protein expression vector Phr-IgG and expressed on HEK293 and Expi-CHO-S. After purification, FACS and endocytic activity validation assays were performed, and a fully human-derived antibody molecule NEC49 was finally selected.

The variable region sequences of the fully human-derived antibody molecule NEC49 are as follows:

heavy chain variable region of NEC49: EVQLLESGGGLVQPGGSLRLSCAASGFTFS SYAMSWVRQAPGKGLE WVSAIYSGGSTYYADSVKGRFTISRDNSKNTLYLQMN SLRAEDTAVYYCTRHGGDSGSW-SYYYYGMDVWGQGTTVTVSS SEQ ID NO: 4

light chain variable region of NEC49:

DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPLTFGQGTRLEIK

SEQ ID NO: 5.

Table 1. CDR sequences obtained by the Kabat numbering scheme

| Antibody | NEC49 | SEQ ID NO |
|---|---|---|
| Heavy chain CDR1 | SYAMS | SEQ ID NO: 8 |

(continued)

| Antibody | NEC49 | SEQ ID NO |
|---|---|---|
| Heavy chain CDR2 | AIYSGGSTYYADSVKG | SEQ ID NO: 9 |
| Heavy chain CDR3 | HGGDSGSWSYYYYGMDV | SEQ ID NO: 10 |
| Light chain CDR1 | RASQSISSYLN | SEQ ID NO: 11 |
| Light chain CDR2 | AASSLQS | SEQ ID NO: 12 |
| Light chain CDR3 | QQSYSTPLT | SEQ ID NO: 13 |

The anti-Nectin4 antibody described above may further comprise an antibody heavy chain constant region and a light chain constant region; the heavy chain constant region may be selected from the group consisting of human IgG1, IgG2, IgG3, and IgG4 constant regions, and the light chain constant region may be selected from the group consisting of human antibody κ and λ chain constant regions. In the present disclosure, the antibody comprises a heavy chain constant region of human IgG1 and a human κ light chain constant region. The sequences of the antibody molecule NEC49 of the present disclosure are as follows:

NEC49 heavy chain:

EVQLLESGGGLVQPGGSLRLSCAASGFTFSSYAMSWVRQAPGKGLEWVSAIYS
GGSTYYADSVKGRFTISRDNSKNTLYLQMNSLRAEDTAVYYCTRHGGDSGSWS
YYYYGMDVWGQGTTVTVSSASTKGPSVFPLAPSSKSTSGGTAALGCLVKDYFP
EPVTVSWNSGALTSGVHTFPAVLQSSGLYSLSSVVTVPSSSLGTQTYICNVNHKP
SNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK

SEQ ID NO: 6

NEC49 light chain:

DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPLTFGQGTRLEIKRTVAA
PSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

SEQ ID NO: 7.

**II. Construction of ADC molecules**

**ADC drug loading determination**

The drug loading of ADCs was determined by reversed-phase high performance liquid chromatography (RP-HPLC).

**1. Determination method:**

A naked antibody and a test sample ADC (at concentration 1 mg/mL) were reduced with 4 μL of DDT (sigma) in a water bath at 37 °C for 1 h, and then transferred to an insert. Analysis was performed on a high performance liquid chromatograph Agilent 1200, with Agilent PLRP-S 1000A 8 μm 4.6 × 250 mm selected as the chromatographic column,

the column temperature at 80 °C, the DAD detector at wavelength 280 nm, the flow rate at 1 mL/min, and the injection volume at 40 μL. Comparisons were made to the spectra of the sample and the naked antibody to identify the locations of the light chain and heavy chain, and then integration was performed on the spectrum of the test sample to calculate the DAR value.

**2. Preparation of solutions**

1) 0.25 M DTT solution:
2) Mobile phase A (0.1% TFA in water):
3) Mobile phase B (0.1% TFA in acetonitrile):

**3. Data analysis**

Comparisons were made to the spectra of the sample and the naked antibody to identify the locations of the light chain and heavy chain, and then integration was performed on the spectrum of the test sample to calculate the drug loading DAR value. The calculation formula is as follows:

| Name | Number of linked drugs |
|---|---|
| LC | 0 |
| LC+1 | 2 |
| HC | 0 |
| HC+1 | 2 |
| HC+2 | 4 |
| HC+3 | 6 |

$$\text{Total LC peak area} = \text{LC peak area} + \text{LC}^{+1} \text{ peak area}$$

Total HC peak area = HC peak area + $HC^{+1}$ peak area + $HC^{+2}$ peak area + $HC^{+3}$ peak area

LC DAR = Σ(number of linked drugs × percent peak area)/total LC peak area HC DAR = Σ(number of linked drugs × percent peak area)/total HC peak area DAR = LC DAR + HC DAR.

**Example 3-1. ADC-1**

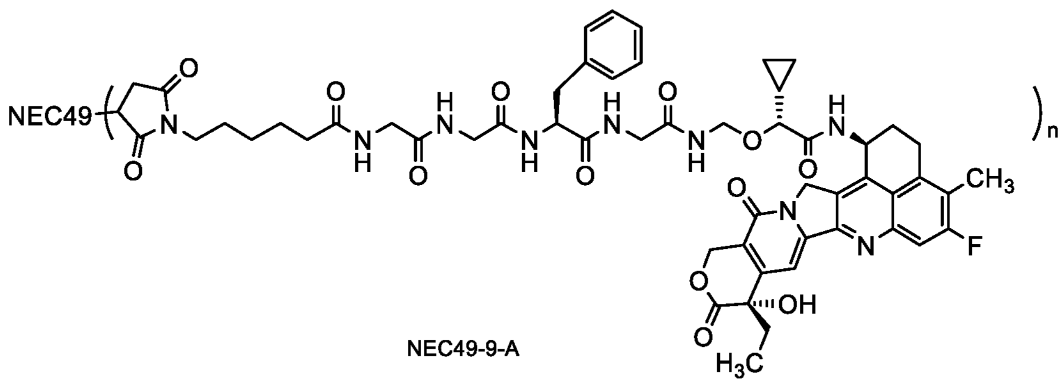

NEC49-9-A

To an aqueous solution of antibody NEC49 in PBS (a pH 6.5, 0.05 M aqueous PBS solution; 10.0 mg/mL, 6.4 mL, 432 nmol), a prepared aqueous solution of tris(2-carboxyethyl)phosphine hydrochloride (TCEP-HCl) (10 mM, 130 μL, 1300 nmol) was added at 37 °C. The mixture was shaken on a water bath shaker at 37 °C for 3 h, and then the reaction was stopped. The reaction mixture was cooled to 25 °C in a water bath.

Compound **9-A** (prepared using the method for compound 9-A of Example 9 on pages 58-60 of WO2020063676, 5.6 mg, 5214 nmol) was dissolved in 300 μL of DMSO, and the resulting solution was added to the above reaction mixture. The mixture was shaken on a water bath shaker at 25 °C for 3 h, and then the reaction was stopped. The reaction mixture

was desalted and purified using a Sephadex G25 gel column (elution phase: a pH 6.5, 0.05 M aqueous PBS solution, containing 0.001 M EDTA) to give an exemplary product of the title conjugate general formula NEC49-9-A, **ADC-1,** in PBS (2.89 mg/mL, 21 mL). The product was then refrigerated at 4 °C. Average loading calculated by RP-HPLC: n = 4.90.

## Example 3-2. ADC-2

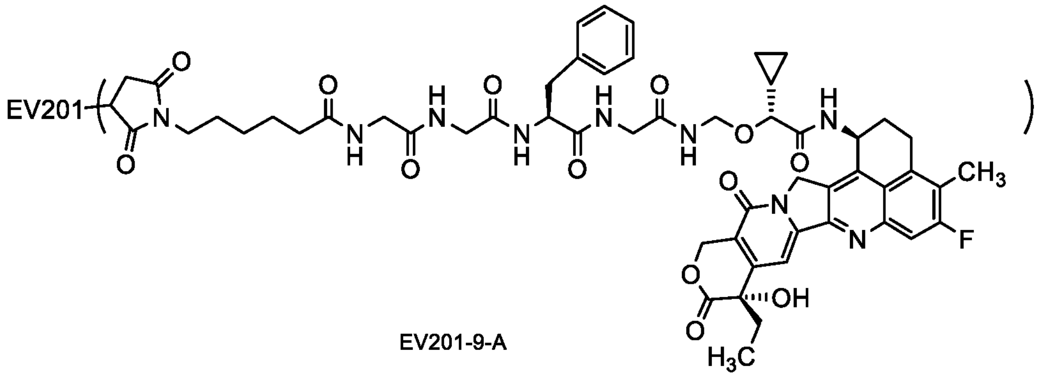

EV201-9-A

To an aqueous solution of control antibody EV201 in PBS (a pH 6.5, 0.05 M aqueous PBS solution; 10.0 mg/mL, 1.63 mL, 110 nmol), a prepared aqueous solution of tris(2-carboxyethyl)phosphine hydrochloride (TCEP-HCl) (10 mM, 28.5 μL, 285 nmol) was added at 37 °C. The mixture was shaken on a water bath shaker at 37 °C for 3 h, and then the reaction was stopped. The reaction mixture was cooled to 25 °C in a water bath.

Compound **9-A** (1.18 mg, 1100 nmol) was dissolved in 90 μL of DMSO, and the resulting solution was added to the above reaction mixture. The mixture was shaken on a water bath shaker at 25 °C for 3 h, and then the reaction was stopped. The reaction mixture was desalted and purified using a Sephadex G25 gel column (elution phase: a pH 6.5, *0.05 M* aqueous PBS solution, containing 0.001 M EDTA) to give an exemplary product of the title conjugate general formula EV201-9-A, **ADC-2,** in PBS (1.12 mg/mL, 12 mL). The product was then refrigerated at 4 °C. Average loading calculated by RP-HPLC: n = 3.90.

## Example 3-3. ADC-3

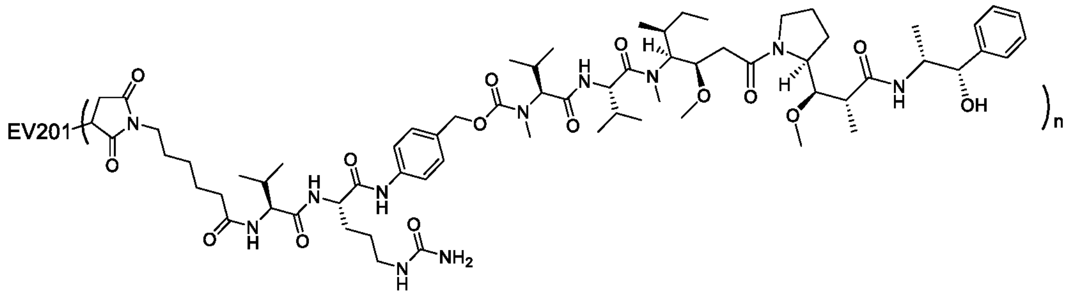

EV201-MMAE

To an aqueous solution of antibody EV201 in PBS (a pH 6.5, 0.05 M aqueous PBS solution; 10.0 mg/mL, 3.67 mL, 248 nmol), a prepared aqueous solution of tris(2-carboxyethyl)phosphine hydrochloride (TCEP-HCl) (10 mM, 64.4 μL, 644 nmol) was added at 37 °C. The mixture was shaken on a water bath shaker at 37 °C for 3 h, and then the reaction was stopped. The reaction mixture was cooled to 25 °C in a water bath.

The compound VcMMAE (Biochempartner, CAS 646502-53-6, 3.3 mg, 2480 nmol) was dissolved in 150 μL of DMSO, and the resulting solution was added to the above reaction mixture. The mixture was shaken on a water bath shaker at 25 °C for 3 h, and then the reaction was stopped. The reaction mixture was desalted and purified using a Sephadex G25 gel column (elution phase: a pH 6.5, 0.05 M aqueous PBS solution, containing 0.001 M EDTA) to give an exemplary product of the title conjugate general formula EV201-MMAE, **ADC-3,** in PBS (2.71 mg/mL, 14 mL). The product was then refrigerated at 4 °C. Average loading calculated by RP-HPLC: n = 4.09.

**Example 3-4. ADC-4**

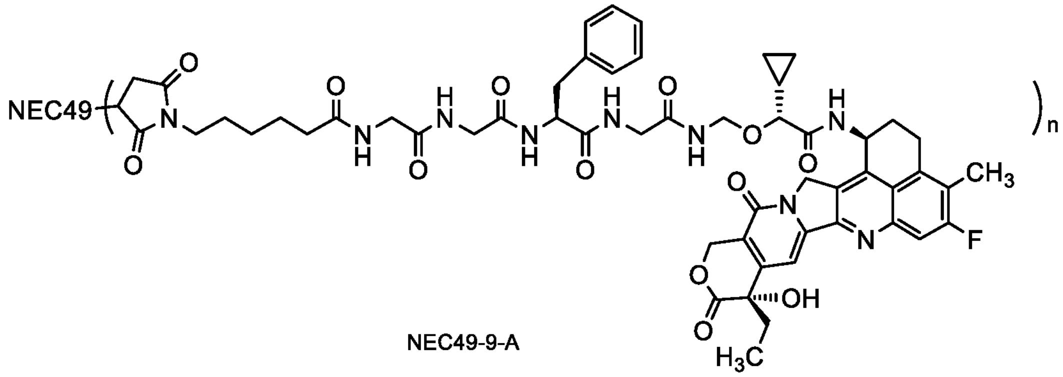

NEC49-9-A

186.37 g of antibody NEC49 (1.28 mmol, diluted with 20 mM histidine-acetic acid to an antibody concentration of 23 mg/mL) and 0.8873 g of tris(2-carboxyethyl)phosphine hydrochloride (Sigma, 3.10 mmol) were stirred in a 20 mM histidine-acetic acid-tris buffer (pH 7.3) containing 2.5 mM EDTA in a thermostatic water bath at 12 °C for 100 min to give a solution of intermediate I. 2 M acetic acid was added dropwise to adjust the pH of the reaction mixture to 5.0.

Compound 9-A (6.13 g, 5.71 mmol) was dissolved in 0.446 L of DMSO to give a solution of compound 9-A in DMSO. 0.365 L of DMSO was added to the above solution of intermediate I in advance, and the above solution of compound 9-A in DMSO was added to the solution of intermediate I to which DMSO had been added in advance. The mixture was stirred at 12 °C for 100 min, and then the reaction was stopped. The product ADC-4 (20 g/L, 175.93 g, average calculated by reversed-phase chromatography: n = 4.0) was obtained.

**Biological Assays**

**Test Example 1. Antibody Protein Level Affinity and Kinetics**

The anti-Nectin-4 antibody was analyzed by Biacore T200 (GE) for characteristic affinity and binding kinetics. A Protein A biosensor chip (Cat. # 29127556, GE) was used to affinity-capture IgG antibodies, and then the human Nectin-4 His (Cat. # 19771-H08H, Sino Biological) antigen that was diluted with HBS-EP buffer (pH 7.4) (Cat. # BR-1001-88, GE) to a series of concentrations was allowed to flow over the surface of the chip. The antigen-antibody binding kinetics was tracked for 3 min and the dissociation kinetics was tracked for 10 min. Reaction signals were detected in real time using a Biacore T200 instrument to obtain binding and dissociation curves. After dissociation was complete in each cycle, the biosensor chip was washed with 10 mM Gly-HCl pH 1.5 (Cat. # BR-1003-54, GE) for regeneration. The data obtained were analyzed with BIAevaluation software of GE using a 1:1 (Langmuir) binding model. The ka (kon), kd (koff) and KD values determined in this way are shown in Table 2-1.

Table 2-1. The affinity of the antibodies determined by Biacore

| Antibody | ka (1/Ms) | kd (1/s) | KD (M) |
|---|---|---|---|
| EV201 | 7.09E+05 | 6.61E-03 | 9.33E-09 |
| NEC49 | 3.67E+05 | 1.89E-03 | 5.15E-09 |

**Test Example 2. *In Vitro* Binding of Antibodies to Cells**

Nectin4-CHOK1/T47D/MDA-MB-468 cells were suspended in FACS buffer (2% fetal bovine serum (Gibco, 10099141) pH 7.4 PBS (Sigma, P4417-100TAB)) to form a 1 $\times$ $10^6$/mL cell suspension, and the suspension was then added to a 96-well round-bottom plate at 100 $\mu$L/well. After centrifugation and removal of the supernatant, the test antibody that was diluted with the FACS buffer to different concentrations was added at 50 $\mu$L/well. The plate was incubated in the dark in a 4 °C refrigerator for 1 h. The plate was washed 3 times with FACS buffer by centrifugation at 500 g, and Alexa Fluor 488 Goat anti-Human IgG (H+L) (invitrogen, A-11013) was then added in a working concentration. The plate was incubated in the dark in a 4 °C refrigerator for 40 min. The plate was washed 3 times with FACS buffer by centrifugation at 500 g and tested on a BD FACSCantoII flow cytometer for geometric mean fluorescence intensity, and the $EC_{50}$ values for the binding of the antibodies to Nectin-4-expressing cells were calculated. The results are shown in Table 2-2.

Table 2-2. The binding activity of the antibodies to different cells

| Cell | Nectin4-CHOK 1 | | T47D | | MDA-MB-468 | |
|---|---|---|---|---|---|---|
| Antibody | NEC49 | EV201 | NEC49 | EV201 | NEC49 | EV201 |
| Maximal fluorescence value | 82484 | 65807 | 13403 | 12638 | 10191 | 9867 |
| $EC_{50}$ (nM) | 0.78 | 0.94 | 0.07 | 0.26 | 0.06 | 0.23 |

Conclusion:

NEC49 has binding activity for a variety of cell lines expressing Nectin-4 and the binding activity is better than that of the control molecule.

**Test Example 3. DT3C Antibody Endocytosis Assay**

This assay aims to examine the endocytosis of the Nectin-4 antibody according to the killing of cells by activated DT after DT3C protein enters the cells. The *in vitro* endocytic activity of the antibodies was evaluated according to $IC_{50}$ and maximal killing values.

DT3C is a recombinantly expressed fusion protein formed by fusing fragment A of diphtheria toxin (toxin moiety only) and fragment 3C of group G streptococcus (IgG binding portion). The protein has a high affinity for the Fc portion of an antibody. It enters cells together with the Fc portion when the antibody is endocytosed, and releases toxic DT under the action of intracellular furin protease. The DT can inhibit the activity of EF2-ADP ribosylation, block the protein translation process and finally cause cell death. DT3C that does not enter the cell has no activity of cell killing. The endocytic activity of the antibody was evaluated according to cell killing.

A Nectin4--CHOK1 cell suspension was prepared using a fresh cell culture medium containing 20% low IgG FBS at a cell density of $2 \times 10^4$ cells/mL, and added to a cell culture plate 3903 at 50 μL/well. The plate was incubated with 5% carbon dioxide at 37 °C for 16 h. DT3C was diluted to 1.6 μM with serum-free culture medium, and the antibodies were diluted to 266.4 nM with serum-free culture medium. 80 μL of DT3C and 80 μL of antibody were mixed (1: 1, v/v) and incubated at room temperature for 30 min. The molar concentration of DT3C was 6 times that of the antibodies.

The DT3C-antibody mixture was serially diluted 4-fold with serum-free culture medium to 8 concentrations. The 9th and 10th points were pure culture media. C25-IgG1 was an IgG1 isotype negative control group. 50 μL of the diluted mixture was added to 50 μL of cells and incubated in an incubator for three days. To each well, 50 μL of CTG was added. The plate was incubated in the dark at room temperature for 10 min. A white membrane was attached to the bottom of the cell culture plate. The plate was placed on a microplate reader Victor 3, and chemiluminescence readings were taken. The results are shown in Table 3.

Table 3. *In vitro* endocytic activity of antibodies

| Antibody | NEC49 | EV201 |
|---|---|---|
| Maximal killing value | 100.55% | 92.59% |
| $IC_{50}$ (nM) | 0.43 | 0.50 |

**Test Example 4. pHrodo Antibody Endocytosis Assay**

This assay aims to examine the endocytosis of the Nectin-4 antibody according to changes in fluorescence signal following internalization of the dye. The *in vitro* endocytic activity of the antibody was evaluated according to the intensity of the fluorescent signal.

Fab fragments coupled with the pH sensitive pHrodo iFL dye can bind directly to the Fc region of the Nectin-4 antibody without affecting antibody recognition of the antigen. The pHrodo iFL dye hardly fluoresces at neutral pH. When the Nectin-4 antibody is endocytosed, the dye is internalized at the same time. The fluorescence signal will gradually intensify as the pH decreases. The endocytic activity of the antibody was evaluated according to how the fluorescence signal intensified.

Nectin4-CHOK1 clone 3 cells were cultured with DMEM/F12 + 10% FBS + 10 μg/mL puromycin. On the first day of the assay, a $2 \times 10^5$ cells/mL cell suspension was prepared with a fresh cell-containing culture medium and added to a 96-well cell culture plate at 100 μL/well. The plate was cultured at 37 °C in 5% carbon dioxide for 24 h.

4× antibody preparation: The antibody stock solution was diluted 10-fold in FBS-free culture medium to prepare a culture medium solution, and the culture medium solution was diluted to prepare a 4× dosing solution (80 nM), with a final

antibody concentration of 20 nM.

4× Zenon™ pHrodo™ iFL IgG labeling reagent preparation: The pHrodo™ labeling reagent stock solution was diluted with FBS-free culture medium to 4× concentration (240 nM), with a final concentration of 60 nM. The above 4× antibody solution and 4× pHrodo™ labeling reagent solution were mixed together in equal volumes and incubated at room temperature for 10 min. 50 μL of the mixture was added to 150 μL of serum-free culture medium, so that each antibody sample had two concentrations (20 nM and 5 nM).

50 μL of the cell broth was removed from the 3903 plate, and 50 μL of a mixture of antibody and pHrodo dye was added to each well. Two replicate wells were set for each antibody sample. A dye addition-only group and an isotype IgG1 control group were set. After 24 hours of culture in an incubator, the culture medium was removed, and the cells in each well were digested with 50 μL of trypsin for 2 min. The digestion was stopped with 50 μL of fresh culture medium. The cells from the replicate wells of the same sample were transferred to a well of a round-bottom plate using a multi-channel pipette. The cells were centrifuged at 1500 rpm for 2 min, and the culture medium was discarded. The cells were washed once with FACS buffer (PBS + 2.5%FBS) and centrifuged at 1500 rpm for 2 min. The cells were resuspended in 200 μL of FACS buffer (PBS + 2.5% FBS), and FITC signals were detected using a flow cytometer. Data was analyzed by Flowjo 7.6. The endocytosis results of NEC49 and EV201 on Nectin4-CHOK1 clone3 (C25-IgG1 as IgG1 isotype negative control) are shown in Table 4.

Table 4. *In vitro* endocytic activity of antibodies

| Sample | 20nM Ab+60nM pHrodo | 5nM Ab+15nM pHrodo |
|---|---|---|
| | FITC-A signal value | FITC-A signal value |
| NEC49 | 1084 | 303 |
| EV201 | 862 | 281 |
| C25-IgG1 | 154 | 129 |
| pHrodo, antibody-free | 160 | 127 |
| Culture medium | 11 | 131 |

The pHrodo assay shows that NEC49 has endocytic activity on Nectin4-CHOK1 cells and the endocytic activity is better than that of the control molecule.

**Test Example 5. Cell Activity Assay of ADC Molecules**

This assay aims to examine the killing effects of Nectin4-ADC samples on cells and to evaluate the *in vitro* activity of Nectin4-ADC according to $IC_{50}$ and maximal kill values. T47D (human mammary gland ductal carcinoma cell, ATCC® HTB-133™), MDA-MB-468 (human breast cancer cell, ATCC® HTB-132™) and MDA-MB-231 (human breast cancer cell, ATCC® HTB-26™) cells were digested with trypsin, neutralized with fresh culture medium, centrifuged at 1000 rpm, and then resuspended in culture medium. After counting, the cell suspension was adjusted to a density of 3703 cells/mL and added to a 96-well cell culture plate 3903 at 135 μL/well. Cells were not added to column 11, and only 135 μL of culture medium was added. The plate was incubated at 37 °C in 5% carbon dioxide for 16 h.

With the concentration of the stock solutions of the samples as the initial concentration, ADC-1 and ADC-2 were diluted five-fold with PBS to 8 concentrations. The cell culture plate was taken out, and 15 μL of the 10× solution was added to each well. The plate was cultured at 37 °C with 5% carbon dioxide for 6 days.

To each well, 70 μL of CTG was added. The plate was incubated in the dark at room temperature for 10 min. A white membrane was attached to the bottom of the cell culture plate. The plate was placed on Victor3, and chemiluminescence readings were taken. The data from this assay were processed using the data processing software GraphPad prism 5.0. The results are shown in Table 5.

Table 5. The killing of cells by Nectin4-ADC drugs

| **Sample name** | T47D (+++) $IC_{50}$ (nM) | MDA-MB-468 (++) $IC_{50}$ (nM) | MDA-MB-231 (-) $IC_{50}$ (nM) |
|---|---|---|---|
| ADC-1 | 111.20 | 87.52 | 1758.00 |
| ADC-2 | 192.60 | 151.50 | 967.60 |
| Note: + represents the expression level of Nectin4 on cells: the more +, the higher the expression level. | | | |

NEC49-9-A (ADC-1) showed a significant killing effect in cells with moderate-to-high expression, and the killing was significantly lower in cells with low expression and no expression. This indicates that the molecule has higher selectivity and medication safety.

**Test Example 6. *In Vivo* Efficacy Evaluation of ADC Molecules in High-Expression CDX Model**

Estrogen tablets (0.36 mg/tablet) were subcutaneously inoculated into the left back of each Balb/c nude mouse. After three days, 0.2 mL ($10 \times 10^6$) of T47D cells (with matrigel, 1:1 (v/v)) were subcutaneously inoculated into the right back of each mouse. When the mean tumor volume reached about 150 $mm^3$, mice were divided into 5 groups of 8 and administration was started.

Three doses of an ADC compound (prepared in PBS) were intravenously injected into each mouse at 10 μL/g body weight. The blank solvent group was injected with PBS.

The tumor volumes and body weights were measured twice a week, and the data were recorded. Data were recorded using Excel statistical software: averages were calculated using avg; SD values were calculated using STDEV; SEM values were calculated using STDEV/SQRT (number of animals per group); plots were generated using GraphPad Prism software, and statistical analysis of data was performed using Two-way ANOVA or One-way ANOVA.

Tumor volume (V) was calculated using the formula: $V = 1/2 \times L_{long} \times L_{short}^2$

$$\text{Relative tumor proliferation rate T/C (\%)} = (T - T_0)/(C - C_0) \times 100\%,$$

where T and C represent the tumor volumes of the treatment group and the control group at the end of the experiment; $T_0$ and $C_0$ represent the tumor volumes at the beginning of the experiment.

$$\text{Tumor growth inhibition rate TGI (\%)} = 1 - \text{T/C (\%)}.$$

The results are shown in Table 6.

Table 6. The efficacy of ADCs against T47D xenograft tumors in tumor-bearing nude mice

| ADC | Tumor growth inhibition rate TGI (%) | |
|---|---|---|
| | 5 mpk | 2.5 mpk |
| ADC-3 | 101 | 73 |
| ADC-1 | 121 | 97 |

In this assay, the test samples ADC-3 and ADC-1 showed significant inhibitory effects on the growth of the xenograft tumors of human breast cancer T47D cells in mice, and the effects were significantly dose-dependent. ADC-1 has a better inhibitory effect on tumor growth than the control molecule in identical doses.

**Test Example 7. Toxicity of ADC Molecules in Rats**

Three doses (one dose per week) of an ADC compound (prepared in normal saline) were intravenously injected into the tail of each SD rat (male, Zhejiang Vital River Laboratory Animal Technology Co., Ltd.) from groups of 4 at 5 mL/kg body weight. Experimental design:

Table 7. Administration regimen for toxicity testing

| Group | ADC compound | Dose of administration (mg/kg) |
|---|---|---|
| Vehicle | Normal saline | 0 |
| A | ADC-3 | 50 |
| B | ADC-1 | 50 |

The results show that: in group A, animal death, cessation of eating, and rapid body weight loss occurred after the first administration; the experiment was immediately stopped, and adrenomegaly and intussusception were observed upon dissection. In group B, following three consecutive administrations, no abnormalities were found during clinical

animal observations, upon dissection and in the blood coagulation function. The results are shown in Table 8.

Table 8. The toxicity test results for ADC compounds in SD rats

| Indicators of abnormality | Hematological index (mean ± SD) | | | Blood biochemical index (mean ± SD) | | Organ index (%, mean ± SD) | |
|---|---|---|---|---|---|---|---|
| 50mpk | WBC (*$10^9$/L) | RET (*$10^9$/L) | LYM (*$10^9$/L) | ALT (IU/L) | TBIL (μmol/L) | Thymus | Liver |
| Normal saline | 10.6±0.2 | 306.6±12.1 | 9.5±0.3 | 31.9±3.6 | 0.6±0.4 | 0.2±0.02 | 3.3±0.7 |
| ADC-3 | 2.7±0.7 | 49.7±14.5 | 2.5±0.7 | 422.8±51.0 | 36.1±11.0 | 0.2±0.01 | 4.5±0.04 |
| ADC-1 | 9.8±1.8 | 273.9±52.6 | 8.2±1.5 | 44.5±7.3 | 0.3±0.1 | 0.07±0.02 | 3.2±0.07 |
| Note: WBC (white blood cells), RET# (reticulocytes), LYM (lymphocytes), ALT (glycine aminotransferase), TBIL (total bilirubin) | | | | | | | |

Conclusion:

The ADC-3 group showed animal death after the first administration and abnormalities in several hematological indexes and blood biochemical indexes. After the ADC-1 group finished three consecutive administrations, no abnormalities were found during clinical animal observations, upon dissection and in the blood coagulation function, and no significant abnormalities were detected in these indexes except for a significant reduction in the size of the thymus.

The preliminary results of the toxicity test in rats suggest that ADC-1 is safer *in vivo* than the control molecule in that it has lower toxic and side effects.

**Test Example 8. Pharmacokinetics of ADC Molecule**

One injection was intravenously administered to each F344 RG rat (male, Beijing Vital-star Biotechnology Co., Ltd.) from groups of 4 at 5 mL/kg body weight, 10 mg/kg body weight. 0.2-mL whole blood samples were collected before the administration and at 5 min, 8 h, 1 d, 2 d, 4 d, 7 d, 10 d, 14 d, 21 d and 28 d after the administration, left at 4 °C for 30 min (without anticoagulation), and centrifuged at 1000 g for 15 min. The supernatants (serum) were placed into EP tubes and stored at -80 °C. The antibody (total antibody) concentration and intact ADC concentration in the serum were determined by Elisa. The results are shown in Table 9. Experimental results:

Table 9. The PK half-life and primary pharmacokinetic parameters in F344 RG rats

| ADC-1 10mpk | Total antibody | Intact ADC |
|---|---|---|
| T1/2 (days) | 3.5 ± 0.7 | 3.1 ± 0.6 |
| AUC 0-t (ug/mL*h) | 18998.27 | 18176.38 |
| Cmax (ug/mL) | 346.11 | 380.57 |
| CL (mL/day/kg) | 12.41 | 13.15 |
| AUC intact ADC/total antibody | 95.7% | |
| ADC-1 has relatively good plasma stability in rats. | | |

**III. Formulations**

**The equipment used in the formulation preparation and determination and the calculation method for results are shown below:**

**1) SEC molecular exclusion chromatography:**

This is a method for analyzing the separation of a solute by the relative relationship between the pore size of the gel pores and the size of the polymer sample molecule coil. SEC% (SEC monomer content percentage) = A monomer/A total × 100% (A monomer represents the peak area of the main peak monomer in the sample, and A total represents the sum of all peak areas.)

$^{\Delta}$SEC% represents the difference between the test item after the sample was left to stand under the conditions and the test item at the start of the standing.

Instrument for SEC measurement: Agilent 1260; column: waters, XBrige BEH200Å SEC (300 × 7.8 mm 3.5 μm).

**2) R-CE capillary gel electrophoresis:**

This is a method of moving the gel into a capillary as a supporting medium for electrophoresis and performing separation according to the molecular weight of the sample under a certain voltage.

R-CE purity percentage = A main peak/A total × 100% (A main peak represents the light chain main peak area + the heavy chain main peak area, and A total represents the sum of all peak areas).

Instrument for R-CE determination: Beckman model plus800

$^{\Delta}$R-CE% represents the difference between the test item after the sample was left to stand under the conditions and the test item at the start of the standing.

**3) Turbidity measurement:**

The degree to which light is hindered from passing through a water layer indicates the ability of the water layer to scatter and absorb light. It is related not only to the suspended matter content but also to the particle composition, size and shape as well as the reflection performance of its surface. In comparing the absorption values of the same protein sample at the same concentration at the same wavelength (within near ultraviolet and visible wavelength regions), the greater the absorption value is, the greater the turbidity is, the more the protein molecules in the sample tend to aggregate. The instrument for measurement was a multifunctional microplate reader (Molecular Devices M5). Samples in the same volume were added to a 96-well plate and the absorbance values were read.

**4) Osmotic pressure determination:**

The freezing point method is used for determining the osmotic pressure. On the basis of the directly proportional relation between the freezing point depression value and the molar concentration of the solution, the freezing point of a solution is determined by using a high-sensitivity temperature-sensing element, and then converted into the osmotic pressure through electric quantity. Manufacturer of instrument: Loser, model: OM815.

**5) Protein concentration determination:**

In the present disclosure, the concentration of the antibody-drug conjugate is expressed in terms of the concentration of the protein, i.e., the concentration of the antibody moiety in the antibody-drug conjugate.

Since the toxin in antibody-drug conjugate absorbs at the protein characteristic absorption wavelength of 280 nm, and the toxin also absorbs at 370 nm, the following formula was used to calculate the above protein concentration:

$$A_{280nm} = \left(C_{drug} \times E_{drug-280} + C_{mAb} \times E_{mAb-280}\right) \times l$$

$$A_{370nm} = C_{drug} \times E_{drug-370} \times l$$

$$\text{Let} \quad R = \frac{E_{drug-370}}{E_{drug-280}}$$

Namely:

$$C_{mAb} = \frac{R \times A_{280nm} - A_{370nm}}{R \times E_{mAb-280}}$$

In the formula,

$A_{280nm}$: the average absorbance of a single sample of the test sample solution at a wavelength of 280 nm with an optical path length of 1 cm;

$A_{370nm}$: the average absorbance of a single sample of the test sample solution at a wavelength of 370 nm with an

optical path length of 1 cm;
$E_{mAb-280}$: the mass extinction coefficient of the protein at the wavelength of 280 nm, which is 1.532 $g^{-1}cm^{-1}L$;
$E_{drug-280}$: the mass extinction coefficient of the toxin at the wavelength of 280 nm, which is 5.17 $g^{-1}cm^{-1}L$;
$E_{drug-370}$: the mass extinction coefficient of the toxin at the wavelength of 370 nm, which is 17.89 $g^{-1}cm^{-1}L$;
R: the ratio of the extinction coefficient of the toxin at 370 nm to that at 280 nm, which is 3.46;
$C_{mAb}$: the concentration of the protein, mg/mL;
1: the optical path length, cm (here the optical path length is 1 cm).

If the test solution is diluted, the protein concentration is: C (mg/mL) = $C_{mAb} \times N$, and N is a dilution factor.
Instrument for protein concentration determination: ultraviolet-visible spectrophotometer, model: Nano Drop 2000.

**Formulation Example 1. pH and Buffer System Screening**

Formulations containing the buffer systems shown in Table 10, 20 mg/mL ADC-4 (on a protein concentration basis), and 0.4 mg/mL polysorbate 80 (PS80) were prepared. Samples were subjected to a forced degradation study (standing at 40 °C for one month), and the effects of different buffer systems on protein stability were evaluated based on SEC and R-CE.
The results are shown in Table 10. The SEC and R-CE data show that after standing at 40 °C for one month, the formulation containing His-HCl (pH 6.0) showed better monomer purity than the other groups.

Table 10. pH and buffer system screening results

| Group | Buffer system | $^{\Delta}$SEC% | $^{\Delta}$R-CE% |
|---|---|---|---|
| 1 | 10 mM His-HCl pH 5.5 | 2.6 | 3.5 |
| 2 | 10 mM His-HCl pH 6.0 | 2.2 | 2.8 |
| 3 | 10 mM His-HCl pH 6.5 | 3.1 | 3.0 |
| 4 | 10 mM SA pH 5.0 | 5.1 | 6.0 |
| 5 | 10 mM SA pH 5.5 | 4.2 | 3.9 |
| 6 | 10 mM CA pH 5.5 | 6.2 | 5.0 |
| 7 | 10 mM PB pH 6.5 | 4.5 | 4.1 |
| Note: His-HCl represents histidine-histidine hydrochloride; CA represents citric acid-citric acid sodium salt; SA represents succinic acid-succinic acid sodium salt; PB represents disodium hydrogen phosphate-sodium dihydrogen phosphate salt; 40 °C M1: standing at 40 °C for one month; these apply hereinafter. | | | |

**Formulation Example 2. Surfactant Screening**

Formulations containing a 10 mM, pH 6.0 His-HCl buffer, 20 mg/mL ADC-4 (on a protein concentration basis), 80 mg/mL sucrose, and the surfactants shown in Table 11 were prepared. Samples were subjected to a forced degradation study (shaking at 300 rpm and 25 °C for 10 days and 5 freeze-thaw cycles + standing at 25 °C for 2 days), and the effects of different surfactants on protein stability were evaluated based on appearance, SEC and R-CE.
The results are shown in Table 11. After forced degradation, the formulation containing 0.2 mg/mL PS80 was clear and transparent and its appearance was better than those of the other groups. The SEC and R-CE data show that there was no significant difference in purity between the formulations containing the different surfactants.

Table 11. Surfactant screening results

| Group | Surfactant | Standing conditions | Appearance | ΔSEC% | $^{\Delta}$R-CE% |
|---|---|---|---|---|---|
| 1 | 0.2 mg/mL PS80 | F/T 5C+25 °CD2 | Transparent and clear | 0.2 | 0.0 |
| | | Shaking D10 | Transparent and clear | 0.7 | 0.0 |
| 2 | 0.4 mg/mL PS80 | F/T 5C+25 °CD2 | Tiny amounts of fine particles | 0.1 | 0.1 |
| | | Shaking D10 | Tiny amounts of fine particles | 0.2 | 0.1 |

(continued)

| Group | Surfactant | Standing conditions | Appearance | ΔSEC% | ΔR-CE% |
|---|---|---|---|---|---|
| 3 | 0.2 mg/mL PS20 | F/T 5C+25 °CD2 | Small amounts of fine particles | 0.1 | 0.3 |
| | | Shaking D10 | Large amounts of fine particles | 0.6 | 0.3 |
| 4 | 0.6 mg/mL PF68 | F/T 5C+25 °CD2 | Fine particles | 0.1 | 0.3 |
| | | Shaking D10 | Clear and transparent | 0.4 | 0.3 |
| Note: PS80 represents polysorbate 80; PS20 represents polysorbate 20; PF68 represents poloxamer 188; shaking D10 represents shaking for 10 days; F/T 5C+25 °C D2 represents 5 freeze-thaw cycles at -25 °C to 2-8 °C + standing at 25 °C for 2 days; these apply hereinafter. | | | | | |

**Formulation Example 3. Buffer System Ionic Strength Screening**

Formulations containing a 10 mM to 30 mM, pH 6.0 His-HCl buffer, 20 mg/mL ADC-4 (on a protein concentration basis), 0.2 mg/mL PS80, and 80 mg/mL sucrose were prepared, as specifically shown in Table 12 below. Samples were subjected to a forced degradation study (shaking at 300 rpm for 10 days), and the effects of different surfactant concentrations on protein stability were evaluated based on post-exchange pH drift, appearance, SEC, and R-CE.
The results are shown in Table 12. After being shaken for 10 days, both the formulations with ionic strengths of 10 mM and 30 mM were transparent and clear in appearance.

Table 12. A buffer system ionic strength screening table

| Group | Buffer system ionic strength | ΔpH after exchange | Appearance | ΔSEC% | ΔR-CE% |
|---|---|---|---|---|---|
| 1 | 10 mM | 0.07 | Transparent and clear | 0.8 | 0.3 |
| 2 | 30 mM | 0.03 | Transparent and clear | 1.2 | 0.2 |

**Formulation Example 4. Buffer System Ionic Strength Screening**

Formulations containing a 10 mM to 50 mM, pH 6.0 His-HCl buffer, 20 mg/mL ADC-4 (on a protein concentration basis), 0.2 mg/mL PS80, and 80 mg/mL sucrose were prepared, as specifically shown in Table 13 below. Samples were subjected to a forced degradation study (standing at 40 °C for 1 month), and the effects of different surfactant concentrations on protein stability were evaluated based on post-exchange pH drift, appearance, SEC, and R-CE.
The results are shown in Table 13. All the formulations with different ionic strengths were transparent and clear in appearance. The SEC and R-CE data show that there was no significant difference in purity between the formulations with different buffer system ionic strengths.

Table 13. Buffer system ionic strength screening

| Group | Buffer system ionic strength | ΔpH after exchange | Appearance | ΔSEC% | ΔR-CE% |
|---|---|---|---|---|---|
| 1 | 10 mM | 0.07 | Transparent and clear | 1.3 | 0.8 |
| 2 | 30 mM | 0.03 | Transparent and clear | 1.3 | 1.1 |
| 3 | 50 mM | 0.01 | Transparent and clear | 1.3 | 0.9 |

**Formulation Example 5. Excipient and Osmotic Pressure Regulator Screening**

Formulations containing a 30 mM, pH 6.0 His-HCl buffer, 20 mg/mL ADC-4 (on a protein concentration basis), and 0.2 mg/mL PS80 were prepared, and the excipients (e.g., sugar) and osmotic pressure regulators in the formulations are shown in Table 14 below.

Table 14. Excipients and osmotic pressure regulators

| Group | Excipient | Osmotic pressure regulator | Osmotic pressure mOsm |
|---|---|---|---|
| 1 | 80 mg/mL sucrose | None | +307 |

(continued)

| Group | Excipient | Osmotic pressure regulator | Osmotic pressure mOsm |
|---|---|---|---|
| 2 | 40 mg/mL sucrose | 9 mg/mL glycine | +294 |
| 3 | 40 mg/mL a,a-trehalose dihydrate | 9 mg/mL glycine | +279 |

Lyophilized formulations: The appearances of the products after lyophilization were examined. The lyophilization procedure is as follows in Table 15:

Table 15. The lyophilization procedure

| Lyophilization process parameters | Set temperature (°C) | Set time (min) | Holding time (min) | Degree of vacuum (Pa) |
|---|---|---|---|---|
| Pre-freezing | 5 | 10 | 60 | N/A |
| Pre-freezing | -45 | 50 | 120 | N/A |
| Primary drying | -20 | 120 | 3000 | 10 |
| Secondary drying | 25 | 60 | 1 | 10 |
| | 25 | 1 | 600 | 1 |

The results are shown in Table 16. The appearances of the powder cakes obtained after lyophilization show that the bottom of the group with the high sucrose concentration severely collapsed, and after the sucrose concentration was reduced to 40 mg/mL and glycine was added, the powder cakes obtained after lyophilization were even and full without collapses. This indicates that after the sucrose concentration was reduced and glycine was added, the time required for the primary drying was shorter than the time required in the case of the formulation with the high sucrose concentration. The formulations containing 9 mg/mL glycine are preferred.

As for the formulations with 9 mg/mL glycine, there was no significant difference in the appearance of the powder cake obtained after lyophilization between the sucrose group and the α,α-trehalose dihydrate group. Sucrose was selected as the excipient, and the formulation was isotonic at the concentration of 40 mg/mL.

Table 16. The lyophilization results for the osmotic pressure regulator screening

| No. | Appearance after lyophilization | How was the reconstitution |
|---|---|---|
| 1 | The bottom severely collapsed | The reconstitution was easy |
| 2 | The surface of the powder cake was flat, and the edge of the bottom was complete | The reconstitution was easy |
| 3 | The surface of the powder cake was flat, and the edge of the bottom was complete | The reconstitution was easy |

**Formulation Example 6. Confirmation of Formulation**

A stock solution containing 20 mg/mL ADC-4 (on a protein concentration basis), 30 mM His-HCl, pH 6.0, 40 mg/mL sucrose, 0.2 mg/mL PS80, and 9 mg/mL glycine was prepared and lyophilized. Samples were subjected to a forced degradation stability study, and the forced degradation stability was evaluated based on SEC and R-CE.

The results are shown in Table 17. After standing at 40 °C for 1 month, there were no significant changes in SEC and R-CE, indicating that the protein has good stability.

Table 17. Forced degradation stability results

| Group | Appearance after reconstitution | SEC% | R-CE% |
|---|---|---|---|
| | | Monomer | Monomer |
| 1 | Clear and transparent | 97.6 | 98.2 |
| | Clear and transparent | 97.7 | 98.3 |

## Claims

1. A pharmaceutical composition, comprising an antibody-drug conjugate and a buffer, wherein the antibody-drug conjugate has a structure represented by formula NEC49-9-A:

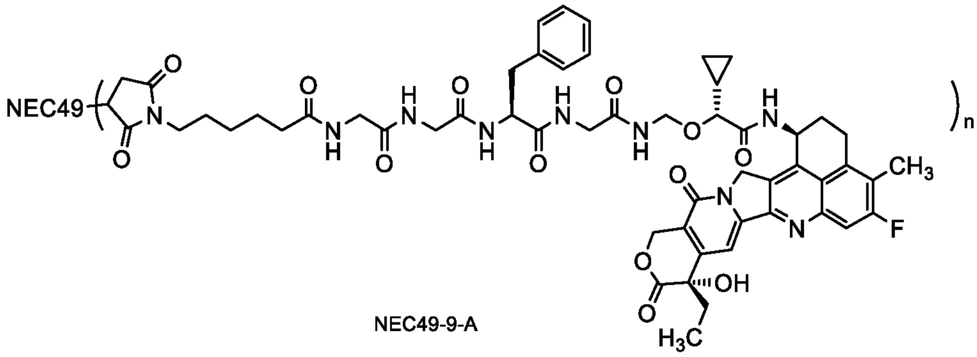

NEC49-9-A

;

wherein:

NEC49 is an anti-Nectin-4 antibody comprising a heavy chain HCDR1, a heavy chain HCDR2, and a heavy chain HCDR3 set forth in SEQ ID NO: 8, SEQ ID NO: 9, and SEQ ID NO: 10; and a light chain LCDR1, a light chain LCDR2, and a light chain LCDR3 set forth in SEQ ID NO: 11, SEQ ID NO: 12, and SEQ ID NO: 13; preferably a heavy chain variable region set forth in SEQ ID NO: 4 and a light chain variable region set forth in SEQ ID NO: 5; and more preferably a heavy chain set forth in SEQ ID NO: 6 and a light chain set forth in SEQ ID NO: 7;
n is a decimal or integer from 1 to 10, preferably from 1 to 8, and more preferably from 3 to 5;
the buffer is histidine-histidine hydrochloride buffer;
the pharmaceutical composition has a pH of about 5.0 to about 6.5, preferably a pH of about 5.5 to about 6.5, and more preferably a pH of about 5.9 to about 6.2.

2. The pharmaceutical composition according to claim 1, wherein the concentration of the buffer is about 10 mM to about 50 mM, preferably about 20 mM to about 40 mM, and more preferably about 30 mM.

3. The pharmaceutical composition according to claim 1 or 2, further comprising a surfactant, wherein the surfactant is preferably polysorbate, more preferably polysorbate 80 or polysorbate 20, and most preferably polysorbate 80.

4. The pharmaceutical composition according to claim 3, wherein the concentration of the surfactant is about 0.05 mg/mL to about 0.4 mg/mL, preferably about 0.1 mg/mL to about 0.3 mg/mL, and more preferably about 0.2 mg/mL.

5. The pharmaceutical composition according to any one of claims 1 to 4, further comprising a sugar, wherein preferably, the sugar is selected from the group consisting of sucrose and $\alpha,\alpha$-trehalose dihydrate; more preferably, the sugar is sucrose.

6. The pharmaceutical composition according to claim 5, wherein the concentration of the sugar is about 25 mg/mL to about 80 mg/mL, preferably about 30 mg/mL to about 50 mg/mL, and more preferably about 40 mg/mL.

7. The pharmaceutical composition according to any one of claims 1 to 6, further comprising an amino acid, wherein the amino acid is preferably glycine.

8. The pharmaceutical composition according to claim 7, wherein the concentration of the amino acid is about 6 mg/mL to about 15 mg/mL, preferably about 7 mg/mL to about 11 mg/mL, and more preferably about 9 mg/mL.

9. The pharmaceutical composition according to any one of claims 1 to 8, wherein the concentration of the antibody-drug conjugate is about 1 mg/mL to about 100 mg/mL on a protein concentration basis;

preferably, the concentration of the antibody-drug conjugate is about 10 mg/mL to about 30 mg/mL on a protein concentration basis;
more preferably, the concentration of the antibody-drug conjugate is about 18 mg/mL to about 22 mg/mL on a

protein concentration basis.

**10.** The pharmaceutical composition according to any one of claims 1 to 9, comprising:

(a) on a protein concentration basis, about 10 mg/mL to about 30 mg/mL said antibody-drug conjugate,
(b) about 0.1 mg/mL to about 0.3 mg/mL polysorbate,
(c) about 30 mg/mL to about 50 mg/mL sucrose or α,α-trehalose dihydrate,
(d) about 7 mg/mL to about 11 mg/mL glycine, and
(e) about 10 mM to about 50 mM histidine-histidine hydrochloride buffer;
the pharmaceutical composition having a pH of about 5.5 to 6.5;
wherein preferably, the pharmaceutical composition comprises:

(a) on a protein concentration basis, about 10 mg/mL to about 30 mg/mL said antibody-drug conjugate,
(b) about 0.1 mg/mL to about 0.3 mg/mL polysorbate 80,
(c) about 30 mg/mL to about 50 mg/mL sucrose,
(d) about 7 mg/mL to about 11 mg/mL glycine, and
(e) about 10 mM to about 50 mM histidine-histidine hydrochloride buffer;

the pharmaceutical composition having a pH of about 5.5 to 6.5;
more preferably, the pharmaceutical composition comprises:

(a) on a protein concentration basis, about 10 mg/mL to about 30 mg/mL said antibody-drug conjugate,
(b) about 0.1 mg/mL to about 0.3 mg/mL polysorbate 80,
(c) about 30 mg/mL to about 50 mg/mL sucrose,
(d) about 7 mg/mL to 11 mg/mL glycine, and
(e) about 20 mM to about 40 mM histidine-histidine hydrochloride buffer;

the pharmaceutical composition having a pH of about 5.5 to 6.5;
most preferably, the pharmaceutical composition comprises:

(a) on a protein concentration basis, about 18 mg/mL to about 22 mg/mL said antibody-drug conjugate,
(b) about 0.2 mg/mL polysorbate 80,
(c) about 40 mg/mL sucrose,
(d) about 9 mg/mL glycine, and
(e) about 30 mM histidine-histidine hydrochloride buffer,

the pharmaceutical composition having a pH of about 5.9 to 6.2.

**11.** A lyophilized formulation comprising an antibody-drug conjugate, capable of being reconstituted to form the pharmaceutical composition according to any one of claims 1 to 10.

**12.** A lyophilized formulation comprising an antibody-drug conjugate, obtained by lyophilizing the pharmaceutical composition according to any one of claims 1 to 10.

**13.** A reconstituted solution comprising an antibody-drug conjugate, prepared and obtained by reconstituting the lyophilized formulation comprising the antibody-drug conjugate according to claim 11 or 12, wherein preferably, the reconstituted solution comprises the following components:

(a) on a protein concentration basis, about 10 mg/mL to about 30 mg/mL said antibody-drug conjugate, (b) about 0.1 mg/mL to about 0.3 mg/mL polysorbate, (c) about 30 mg/mL to about 50 mg/mL sucrose or α,α-trehalose dihydrate, (d) about 7 mg/mL to about 11 mg/mL glycine, and (e) about 10 mM to about 50 mM histidine-histidine hydrochloride buffer; the pharmaceutical composition having a pH of about 5.5 to 6.5;
more preferably, the pharmaceutical composition comprises:

(a) on a protein concentration basis, about 10 mg/mL to about 30 mg/mL said antibody-drug conjugate, (b) about 0.1 mg/mL to about 0.3 mg/mL polysorbate 80, (c) about 30 mg/mL to about 50 mg/mL sucrose, (d) about 7 mg/mL to about 11 mg/mL glycine, and (e) about 10 mM to about 50 mM histidine-histidine hydrochloride buffer;

the pharmaceutical composition having a pH of about 5.5 to 6.5;
most preferably, the pharmaceutical composition comprises:
(a) on a protein concentration basis, about 18 mg/mL to about 22 mg/mL said antibody-drug conjugate, (b) about 0.2 mg/mL polysorbate 80, (c) about 40 mg/mL sucrose, (d) about 9 mg/mL glycine, and (e) about 30 mM histidine-histidine hydrochloride buffer; the pharmaceutical composition having a pH of about 5.9 to 6.2.

**14.** A kit, comprising a container containing the pharmaceutical composition according to any one of claims 1 to 10, the lyophilized formulation comprising the antibody-drug conjugate according to claim 11 or 12, or the reconstituted solution comprising the antibody-drug conjugate according to claim 13.

**15.** Use of the pharmaceutical composition according to any one of claims 1 to 10, the lyophilized formulation comprising the antibody-drug conjugate according to claim 11 or 12, or the reconstituted solution comprising the antibody-drug conjugate according to claim 13 in the preparation of a medicament for treating a Nectin-4-mediated disease or disorder.

**16.** Use of the pharmaceutical composition according to any one of claims 1 to 10, the lyophilized formulation comprising the antibody-drug conjugate according to claim 11 or 12, or the reconstituted solution comprising the antibody-drug conjugate according to claim 13 in the preparation of a medicament for treating a viral infection, a tumor, or a cancer, wherein preferably, the tumor or the cancer is selected from the group consisting of urothelial cancer, ureter cancer, breast cancer, pancreatic cancer, lung cancer, renal cancer, liver cancer, esophageal cancer, a laryngeal tumor, a pharyngeal tumor, an oral tumor, gastric cancer, ovarian cancer, prostate cancer, bladder cancer, colorectal cancer, head and neck cancer, squamous cell carcinoma, and melanoma.

## INTERNATIONAL SEARCH REPORT

International application No.
**PCT/CN2023/094502**

**A. CLASSIFICATION OF SUBJECT MATTER**

C07K 16/28(2006.01)i； C12N 15/13(2006.01)i； A61K 39/395(2006.01)i； A61K 47/68(2017.01)i； A61K 31/4745(2006.01)i； A61P 35/00(2006.01)i

According to International Patent Classification (IPC) or to both national classification and IPC

**B. FIELDS SEARCHED**

Minimum documentation searched (classification system followed by classification symbols)

IPC：C07K，C12N，A61K，A61P

Documentation searched other than minimum documentation to the extent that such documents are included in the fields searched

Electronic data base consulted during the international search (name of data base and, where practicable, search terms used)

VEN, CNABS, PubMed, DWPI, CNTXT, WOTXT, USTXT, EPTXT, JPTXT, CNKI, Web of Science, 百度学术, BAIDU SCHOLAR, Patentics, 中国生物序列检索系统, Chinese Patent Biological Sequence Retrieval System, NCBI Genbank, EBI, STNext: 申请人, 发明人, Nectin-4, Nectin4, Nectin, PVRL4, 偶联物, 序列4-13, Enfortumab vedotin, MMAE, conjugate

**C. DOCUMENTS CONSIDERED TO BE RELEVANT**

| Category* | Citation of document, with indication, where appropriate, of the relevant passages | Relevant to claim No. |
|---|---|---|
| PX | WO 2022228406 A1 (JIANGSU HENGRUI PHARMACEUTICALS CO., LTD. et al.) 03 November 2022 (2022-11-03)<br>claims 2-25 | 1-16 |
| A | CN 110392697 A (INSTITUT NATIONAL DE LA SANTE ET DE LA RECHERCHE MEDICALE et al.) 29 October 2019 (2019-10-29)<br>claims 1-13 | 1-16 |
| A | WO 2020063676 A1 (JIANGSU HENGRUI MEDICINE CO., LTD.) 02 April 2020 (2020-04-02)<br>description, embodiment 9 | 1-16 |
| A | CN 113527486 A (MABWELL (SHANGHAI) BIOTECHNOLOGY CO., LTD.) 22 October 2021 (2021-10-22)<br>entire document | 1-16 |
| A | US 2011301056 A1 (ONCOTHERAPY SCIENCE, INC.) 08 December 2011 (2011-12-08)<br>entire document | 1-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 May 2023** | **07 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/094502**

**C. DOCUMENTS CONSIDERED TO BE RELEVANT**

| Category* | Citation of document, with indication, where appropriate, of the relevant passages | Relevant to claim No. |
|---|---|---|
| A | US 2018243434 A1 (INSERM (INSTITUT NATIONAL DE LA SANTE RECHERCHE MEDICALE) et al.) 30 August 2018 (2018-08-30) entire document | 1-16 |
| A | WO 2021213434 A1 (MABWELL (SHANGHAI) BIOSCIENCE CO., LTD.) 28 October 2021 (2021-10-28) entire document | 1-16 |
| A | 姜根炳等 (JIANG, Genbing et al.). "Nectin-4及上皮间质转化标志物在胃癌组织中的表达 (Expressions of Nectin-4 and Epithelial Mesenchymal Transition-related Proteins in Gastric Cancer)" *江苏医药 (Jiangsu Medical Journal),* Vol. 43, No. 17, 15 September 2017 (2017-09-15), pages 1255-1269 | 1-16 |
| A | TEO, M. Y. et al. "NECTIN4 Heterogeneity and Molecular Diversity in Bladder Cancers: Deconstructing the Activity of An Antibody-Drug Conjugate" *Clinical Cancer Research,* Vol. 27, No. 18, 15 September 2021 (2021-09-15), pages 4950-4952 | 1-16 |

Form PCT/ISA/210 (second sheet) (July 2022)

**INTERNATIONAL SEARCH REPORT**

International application No.
**PCT/CN2023/094502**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/094502**

| Patent document cited in search report | | | Publication date (day/month/year) | Patent family member(s) | | | Publication date (day/month/year) |
|---|---|---|---|---|---|---|---|
| WO | 2022228406 | A1 | 03 November 2022 | None | | | |
| CN | 110392697 | A | 29 October 2019 | EP | 3589654 | A1 | 08 January 2020 |
| | | | | JP | 2020510432 | A | 09 April 2020 |
| | | | | US | 2021324104 | A1 | 21 October 2021 |
| | | | | US | 11274160 | B2 | 15 March 2022 |
| | | | | WO | 2018158398 | A1 | 07 September 2018 |
| WO | 2020063676 | A1 | 02 April 2020 | CA | 3114137 | A1 | 02 April 2020 |
| | | | | JP | 2022511351 | A | 31 January 2022 |
| | | | | BR | 112021004656 | A2 | 01 June 2021 |
| | | | | EP | 3858386 | A1 | 04 August 2021 |
| | | | | EP | 3858386 | A4 | 12 October 2022 |
| | | | | KR | 20210066833 | A | 07 June 2021 |
| | | | | MX | 2021003382 | A | 27 May 2021 |
| | | | | US | 2021353764 | A1 | 18 November 2021 |
| | | | | AU | 2019349207 | A1 | 08 April 2021 |
| | | | | TW | 202027795 | A | 01 August 2020 |
| CN | 113527486 | A | 22 October 2021 | None | | | |
| US | 2011301056 | A1 | 08 December 2011 | WO | 2010067487 | A1 | 17 June 2010 |
| | | | | EP | 2373794 | A1 | 12 October 2011 |
| | | | | EP | 2373794 | A4 | 05 September 2012 |
| | | | | JP | 2012511305 | A | 24 May 2012 |
| US | 2018243434 | A1 | 30 August 2018 | JP | 2018531913 | A | 01 November 2018 |
| | | | | JP | 6985252 | B2 | 22 December 2021 |
| | | | | US | 10675357 | B2 | 09 June 2020 |
| | | | | WO | 2017042210 | A1 | 16 March 2017 |
| | | | | EP | 3347048 | A1 | 18 July 2018 |
| | | | | EP | 3347048 | B1 | 01 April 2020 |
| | | | | ES | 2794557 | T3 | 18 November 2020 |
| WO | 2021213434 | A1 | 28 October 2021 | BR | 112022021322 | A2 | 06 December 2022 |
| | | | | CA | 3176385 | A1 | 28 October 2021 |
| | | | | EP | 4141029 | A1 | 01 March 2023 |
| | | | | AU | 2021260639 | A1 | 08 December 2022 |
| | | | | KR | 20230007406 | A | 12 January 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202210530522
- CN 2022089129 W
- US 20050238649 A1
- US 5208020 A
- WO 2012047724 A
- WO 2020063676 A


### Non-patent literature cited in the description


- **CHARI et al.** *Cancer Research*, 1992, vol. 52, 127-131
- *J. Biol. Chem.*, 1968, vol. 243, 3558
- Antibodies: A Laboratory Manual and Molecular Cloning: A Laboratory Manual. Cold Spring Harbor Laboratory
- *CHEMICAL ABSTRACTS*, 646502-53-6